(19) **Europäisches Patentamt**
**European Patent Office**
**Office européen des brevets**

(11) **EP 1 940 285 B1**

(12) **EUROPEAN PATENT SPECIFICATION**

(45) Date of publication and mention
of the grant of the patent:
**29.05.2019 Bulletin 2019/22**

(51) Int Cl.:
***G16H 50/80*** (2018.01)

(21) Application number: **06815414.5**

(86) International application number:
**PCT/US2006/037387**

(22) Date of filing: **25.09.2006**

(87) International publication number:
**WO 2007/035958 (29.03.2007 Gazette 2007/13)**

(54) **METHOD, APPARATUS AND SOFTWARE FOR IDENTIFYING RESPONDERS IN A CLINICAL ENVIRONMENT**

VERFAHREN, GERÄT UND SOFTWARE ZUR IDENTIFIZIERUNG VON ANSPRECHENDEN PATIENTEN IN EINEM KLINISCHEN UMFELD

PROCEDE, APPAREIL ET LOGICIEL D'IDENTIFICATION DE SUJETS REPONDANTS DANS UN ENVIRONNEMENT CLINIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.09.2005 US 719896 P**

(43) Date of publication of application:
**09.07.2008 Bulletin 2008/28**

(73) Proprietor: **Acorda Therapeutics, Inc.
Ardsley, NY 10502 (US)**

(72) Inventors:
• **COHEN, Ron**
**Irvington, NY 10533 (US)**
• **BLIGHT, Andrew, R.**
**Mahopac, NY 10541 (US)**
• **MARINUCCI, Lawrence**
**White Plains, NY 10606 (US)**

(74) Representative: **Carpmaels & Ransford LLP
One Southampton Row
London WC1B 5HA (GB)**

(56) References cited:
**WO-A2-02/051354      US-A1- 2002 010 552
US-A1- 2003 088 365      US-A1- 2003 143 548
US-A1- 2004 015 337      US-A1- 2004 107 084
US-A1- 2004 172 447      US-A1- 2005 075 832**

• **ZUCKER D R ET AL: "COMBINING SINGLE PATIENT (N-OF-1) TRIALS TO ESTIMATE POPULATION TREATMENT EFFECTS AND THE EVALUATE INDIVIDUAL PATIENT RESPONSES TO TREATMENT" JOURNAL OF CLINICAL EPIDEMIOLOGY, PERGAMON, GB LNKD-DOI:10.1016/S0895-4356(96)00429-5, vol. 50, no. 4, 1 January 1997 (1997-01-01), pages 401-410, XP002952324 ISSN: 0895-4356**
• **WASSMER G ET AL: "Flexible interim analysis in clinical trials using multistage adaptive test designs" DRUG INFORMATION JOURNAL (DRUGS.COM), PERGAMON, NEW YORK, NY, US, vol. 35, no. 4, 1 January 2001 (2001-01-01), pages 1131-1146, XP008119818 ISSN: 0092-8615**
• **DELGADO-HERRERA LETICIA ET AL: "A model for the interim analysis process: a case study" CONTROLLES CLINICAL TRIALS, ELSEVIER SCIENCE LTD., NEW YORK, NY, US LNKD-DOI:10.1016/S0197-2456(02)00275-1, vol. 24, no. 1, 1 February 2003 (2003-02-01), pages 51-65, XP002497624 ISSN: 0197-2456**

**Description**

**BACKGROUND**

Clinical researchers have the ongoing problem of not being able to accurately predict or plan future trials, and are not able to salvage or otherwise learn from failed clinical trials. There are many reasons for this. First, functional measurements from clinical trial subjects with certain kinds of conditions like MS and other ailments can vary extensively and randomly over time. This is problematic, because if these measurements are to be used as treatment outcome measures, the spontaneous variability can obscure the treatment-related effects. This interference between spontaneous and induced changes may be particularly problematic under conditions where only a subset of trial subjects respond to treatment. Under these conditions, the treatment effect in the responsive subjects may be diluted by the non-responders in addition to the contamination of spontaneous variability.

Moreover, clinical trials also frequently rely on just a few, intermittent measurements at widely spaced clinic visits. These few sample measurements will not adequately represent the full range of variation of the outcome variable, either during the baseline comparison period or during the treatment period. Where the magnitude of the spontaneous variability of the population is large compared to the expected treatment effect in the individual, it can be difficult to determine the presence of response to treatment based on the average difference between baseline and treatment periods. A single large outlying value in one direction or the other from the mean may mask a smaller but consistent response or alternatively produce the impression of a response that is not actually consistent during the treatment period. Thus, there is a need for detecting a consistent response to treatment over time without encountering false results from the above-mentioned variables.

It is therefore an object of the invention to provide a means to detect true, consistent response to treatment over time using small numbers of measurements from on-treatment and off-treatment periods has been devised to provide a solution for this problem.

It is further an object of the present invention to provide a method that involves examining the frequency with which values measured during the on-treatment period lie outside the range of values recorded during the off-treatment period(s) of the trial.

WO02051354A2 relates to a system and method for medical researchers into drug procedure or intervention, or device efficacy, safety, economics or use, for developing and testing a decision model that determines for patients individually the probable efficacy, safety, economic benefits and use of drugs or medical devices. The model uses studies to determine the reliability of measurements, criteria of clinical significance, criteria of statistical significance, studies of the internal validity of patient assessments under both double-blind placebo controlled and non-double-blind non-placebo controlled conditions, methods of confirming the predictions from the clinical trial model, and studies of long-term predictions of health status from outcome measurements, and clinical trial or other medical research designs, to identify each individual s response to treatment.

US2005075832A1 relates to a system and method of continuously analyzing trial data of an ongoing clinical trial. A statistical analysis is performed on a trial database containing subject trial data. If the result of the statistical analysis does not exceed a predetermined threshold value, then the statistical analysis is repeated while the clinical trial is ongoing. In a blinded clinical trial, a grouped database is generated from the trial database and a blinding database prior to performing the statistical analysis. The grouped database groups the subject trial data according to the study groups.

**SUMMARY OF THE INVENTION**

The present invention is defined by the independent claims, with optional features of specific embodiments described in the dependent claims.

This invention relates to a method, apparatus, and computer software application that can be used to analyze therapeutic effect of a treatment of patients in a clinical environment.

More specifically, the present invention may be utilized to analyze the response of patients in a clinical environment for many different types of afflictions, including, but not limited to, neurological disorders such as multiple sclerosis, spinal cord injuries, Alzheimer's disease and ALS.

One aspect of the present disclosure relates to a method, apparatus and software program for analyzing clinical patient treatment data in order to predict future clinical trials.

Another aspect of the present disclosure relates to a method, apparatus and software program for analyzing clinical patient treatment data in order to derive value from completed clinical trials, regardless of the outcome of the particular trial.

Another aspect of the present disclosure relates to a method, apparatus and software program for selecting individuals based on responsiveness to a treatment. The method comprises identifying a plurality of individuals; administering a test to each individual prior to a treatment period; administering a treatment to one or more of the individuals during the treatment period; administering the test a plurality of times to each individual during the treatment period; and selecting one or more individuals, wherein the selected individuals exhibit an improved performance during a majority of the tests administered during the treatment period as compared to the test administered prior to the treatment period. In certain aspects, the method may further comprise administering the test to each individual after the treatment period, wherein the selected individuals further exhibit an improved performance during a majority of the tests administered during the treatment period as compared to the test administered after the treatment period.

A further aspect relates to a method of selecting individuals based on responsiveness to a treatment, the method comprising identifying a plurality of individuals; administering a test to each individual prior to a treatment period; administering a treatment to one or more of the individuals during the treatment period; administering the test a plurality of times to each individual during the treatment period; administering the test to each individual after the treatment period; and selecting one or more individuals, wherein the selected individuals exhibit an improved performance during a majority of the tests administered during the treatment period as compared to the better performance of the test administered prior to the treatment period and the test administered after the treatment period.

## BRIEF DESCRIPTION OF THE DRAWINGS

**Figure 1** is an exemplary flow diagram showing one way in which the inventive process may be put forth in a computer aided embodiment so treatment data from a clinical trial of a given number of patients may analyzed to determine the responders therein;

**Figure 2** is an exemplary flow diagram showing one way in which the probability distribution generation of the inventive process may be put forth in a computer aided embodiment in order to offer a comparative baseline against responder values;

**Figure 3** is a generalized system level block diagram of an exemplary system employing the inventive process described herein; and

**Figures 4 (a) - 4 (d)** are histograms and distribution graphs of responder and non-responder populations shown in the context of the an illustrative utilization of the present invention.

## DETAILED DESCRIPTION OF THE INVENTION

Before the present compositions and methods are described, it is to be understood that this invention is not limited to the particular molecules, compositions, methodologies or protocols described, as these may vary. It is also to be understood that the terminology used in the description is for the purpose of describing the particular versions or embodiments only, and is not intended to limit the scope of the present invention which will be limited only by the appended claims.

The terms used herein have meanings recognized and known to those of skill in the art, however, for convenience and completeness, particular terms and their meanings are set forth below.

It must also be noted that as used herein and in the appended claims, the singular forms "a", "an", and "the" include plural reference unless the context clearly dictates otherwise. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. All publications mentioned herein are incorporated by reference. Nothing herein is to be construed as an admission that the invention is not entitled to antedate such disclosure by virtue of prior invention.

"Software" means all forms of electronically executable code, regardless of the language employed for coding, specific system architecture coded for, and regardless of storage medium utilized (disk, download, ASP, etc.).

The terms "patient" and "subject" mean all animals including humans. Examples of patients or subjects include humans, cows, dogs, cats, goats, sheep, rats, pigs, etc.

One aspect of the invention therefore relates to a process of providing for the above mentioned frequency to be compared between treatment and control groups, as well as with the predictions of a simple computer model based on random number generation. Hence, if there are $j$ measurements made during treatment and $k$ measurements made during the non-treatment period, a computer model can be generated that will predict the frequency with which a given subset of the $j$ measurements will exceed the largest of the $k$ off treatment measurements. This is effectuated by using the method and the computer program of the present invention to generate many thousands of strings of $j+k$ random numbers within a preset range and testing the frequency with which numbers in the $j$ set exceed all numbers in the $k$ set. Over the course of many thousand iterations, it will be possible to determine the probability that 1,2,3...$j$ of the $j$ set will exceed all the *k* set within any one iteration.

By way of just one illustration, when *j* and *k* are small integers (say, >3,<8) there will be a relatively high probability that just (or at least) one of the *j* set exceeds the maximum of the *k* set, but the probability will decrease rapidly for higher numbers of the *j* set, with the least probability that all of the numbers in the *j* set will be higher than the maximum of the *k* set. As such, the model will then be able to generate a probability distribution for the number of *j* on-treatment measurements that are likely to exceed the maximum of the *k* off-treatment measurements. The clinical trial data for each individual subject or patient *P* can be examined directly for the number of on-treatment measurements that exceed the maximum off-treatment measurement. The distribution of the number of *j* measurements that exceed the maximum *k* measurement for individuals in the treated group may then be compared with the similar distribution for the placebo-treated or other comparator group.

Differences in the distribution should then be present for the higher numbers of *j* measurements that exceed the maximum *k* measurement. These differences allow a suitable criterion to be established for the minimum number of *j* values exceeding the maximum *k* value that represents a high likelihood of a treatment response, based on a clear separation of probability in the upper part of the range. A working criterion would be that a treatment response is likely where a majority of the *j* measurements exceed the maximum *k* measurement, under the condition that *j* and *k* are closely matched small integers (plus or minus one). The probability that the majority of *j* values lie above the range of *k* values should be low based on random variability.

Similarly, the clinical trial data may also be compared to the probability distribution from the computer model to check that the probability distribution of the comparator data is similar to the random number model and that there is not a profound deviation from the predictions of the model that would indicate a treatment-period related effect that was independent of treatment.

Once the criterion for response can be established by comparison of the treated and comparator distributions, then in subsequent studies this criterion can be used to identify the numbers of people who appear to respond to treatment in the actively treated and comparator or placebo-treated groups and the significance of differences in response rate can be determined by straightforward statistical testing of those frequency. When configured as such, the characteristics of the response to treatment of the responder group can also then be examined, undiluted by the non-responder population. Nevertheless, as can be appreciated, the above descriptions regarding such particulars like the specific comparators employed, the number and type of tests employed, the number or patients, the number of off- and on-treatments may all be modified to suit the particular needs of the clinician and to the specific affliction and/or drug being examined.

Thus, as seen in one exemplary aspect of the disclosure, the broadest aspect of the invention may be detailed as comprising a method, a method instantiated or executed on an electronic apparatus such as a computer, and/or a computer readable medium executing the following steps of: identifying a plurality of records relating to patients in a clinical database, said records comprising measurements for patients relating to tests administered during an off-treatment period and an on-treatment period; identifying at least one test in said plurality of records relating to measurements of each individual during an off-treatment period; identifying at least one test in said plurality of records relating to measurements of each individual during an on-treatment period; identifying a baseline measurement of each individual during said off-treatment period; performing a statistical distribution on said plurality of records to identify likelihood of said on-treatment and said off-treatment measurements exceeding said baseline so as to compare said measurements with said baseline; and selecting one or more individuals ("responders"), wherein the selected individuals exhibit an improved performance during a majority of the tests administered during the on-treatment period as compared to a best (e.g., fastest, strongest, etc.) response the test administered to the off-treatment period. However, as can be appreciated, the invention may take the form of a computer readable medium for executing the above detailed steps, or alternatively, may comprise a computer based system for selecting individuals based on responsiveness to a treatment, comprising:

a memory module for storing patient measurements, and for storing at least a first set of instructions relating to the inputting and analyzing of said patient measurements, and a second set of instructions for outputting responder information from said patient measurements;
a central processing unit for executing said first and second set of instructions; and an output module for outputting said responder information.

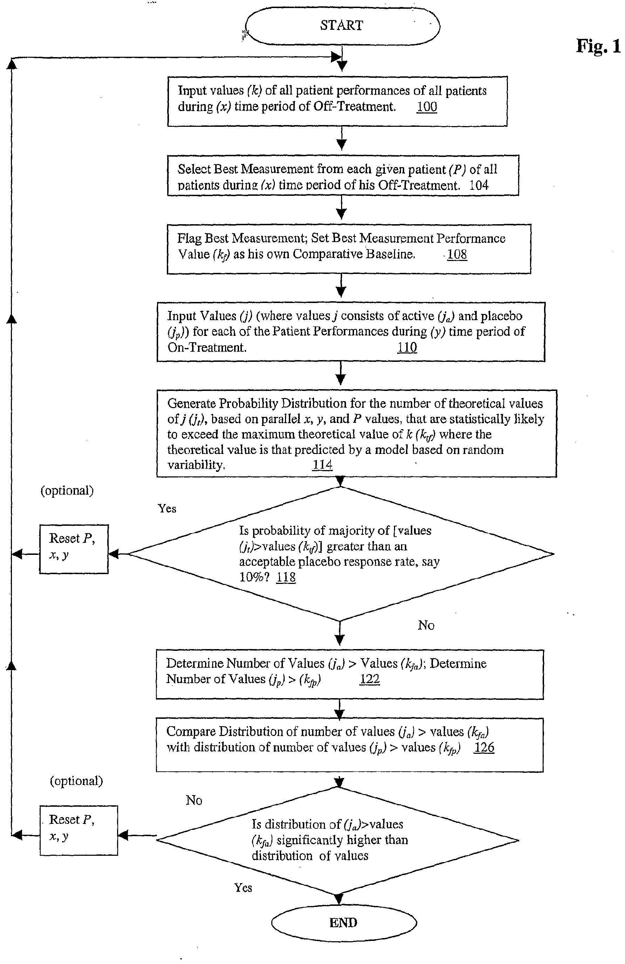

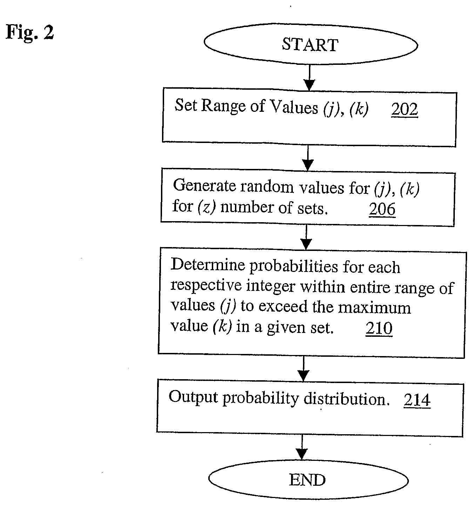

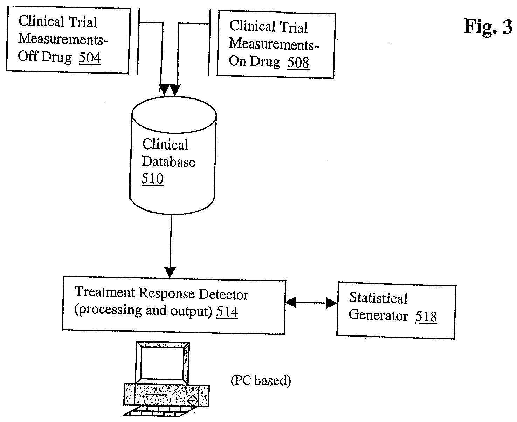

Accordingly, as seen in Figs. 1, 2, and 3, is an exemplary depiction of the inventive process in: a generalized flow diagram (Fig. 1)(showing steps 100 through 130, with optional resets for re-designing or re-conducting the process so as to reset undesirable results); a generalized flow diagram on one approach to generating a specialized, unique statistical distribution (e.g., step 114 of Fig. 1) used within the overall process (e.g., steps 100-130) in Fig. 1; and an exemplary hardware (apparatus) configuration (Fig. 3), upon which the exemplary flow processes in Figs. 1 and 2 are executed by the inventive software. The inventive software for executing the above described processes, and for analyzing inputted data, outputs useful information such as responder data. The inventive software and process may be embodied in computer any manner of readable code, and may be contained on any computer readable medium, such as a hard drive (whether PC based, or remote server), disk, CD, etc. When configured as such, the measurements of the patients P are formatted as signals that may be received by the apparatus of Figure 3 so that the inventive process and software may be transformed into useful outputs for a user. This outputted information may be received by the apparatus in order to be processed and analyzed by the inventive process for use by a user who may receive the outputted signals that have been formed by the steps described herein. As such, the technical effect is such that when the signals are processed in accordance with the above, the tangible, useful result is that clinical trials may be better planned and/or analyzed by researchers who may identify responders to a given treatment for an affliction of almost any nature in ways that were not available heretofore. In any case, in order to achieve this desirable result, it is to be emphasized that the included figures are merely illustrative, and may be reconfigured or revised in many different ways, as one skilled in the art may appreciate.

In a specific exemplary application of one aspect of the present disclosure, a method of analyzing the treatment of an illustrative affliction, such as multiple sclerosis is provided. In such an example, the goal might be to employ the general inventive process and software described herein to show the results of a completed clinical study, or otherwise structure a future clinical study that aims to identify responders from a group of patients who receive a given exemplary treatment. In doing so, many indicators may be employed, but in the exemplary illustration indicated in the attached *Appendices A, B, C, D* and E (each of which is hereby explicitly incorporated by reference in their entireties), such indicators may be such specific measurements as increased walking speed in patients, or increased muscle tone or muscle strength in patients.

Thus, in the given exemplary affliction and clinical treatment depicted in *Appendices A, B, C, D,* and *E*, only a proportion of MS patients would typically be expected to have axons of appropriate functional relevance that are susceptible to these drug effects, given the highly variable pathology of the disease. Nevertheless, when the inventive process and software is employed in the manner described herein, and as broadly illustrated in Figs. 1, 2, and 3, the innovative methodology identifies and characterizes the subset of patients who respond to the exemplary drug fampridine.

To this end, the present invention provides for a method of selecting individuals based on responsiveness to a treatment. In one aspect, the method comprises identifying a plurality of individuals; administering a test to each individual prior to a treatment period; administering a treatment, including, but not limited to administering a therapeutic agent or drug, to one or more of the individuals during the treatment period; administering the test a plurality of times to each individual during the treatment period; and selecting one or more individuals, wherein the selected individuals exhibit an improved performance during a majority of the tests administered during the treatment period as compared to the test administered prior to the treatment period. In certain aspects, the method may further comprise administering the test to each individual after the treatment period, wherein the selected individuals further exhibit an improved performance during a majority of the tests administered during the treatment period as compared to the test administered after the treatment period.

It is important to note that this aspect selects subjects who show a pattern of change that is consistent with a treatment response, but does not define the full characteristics of that response. The criterion itself does not specify the amount of improvement nor does it specify that the improvement must be stable over time. For example, a progressive decline in effect during the course of the study period, even one resulting in speeds slower than the maximum non-treatment value, would not be excluded by the criterion; as a specific example, changes from the maximum non-treatment value of, respectively, +20%, +5%, + 1% and -30% during the double blind treatment period would qualify as a response under the criterion, but would actually show a net negative average change for the entire period, poor stability and a negative endpoint. Post-hoc analyses of studies discussed in greater detail below indicate that we may expect responders defined by consistency of effect also to demonstrate increased magnitude and stability of benefit. Thus, as indicated in *Appendices A, B, C, D,* and *E*, the existence of a subset of patients who respond consistently to the drug can be supported by quantitative observations in the exemplary clinical studies discussed below.

As further noted in the exemplary application of the inventive process and software on the illustrative clinical trial described in *Appendices A, B, C, D,* and *E*, before treatment, the subjects in these two trials exhibited average walking speeds on the TW25 measure of approximately 2 feet per second (ft/sec). This is a significant deficit, since the expected walking speed for an unaffected individual is 5-6 ft/sec. Subjects in MS-F202 were selected for TW-25 walking time at screening of 8-60, which is equivalent to a range in speed of 0.42-3.1 ft/sec. Variability of functional status is an inherent characteristic of MS, and this can be seen in repeated measurement of walking speed over the course of weeks or months. At any of the three visits during the stable treatment period, 15-20% of placebo-treated subjects showed >20% improvement from baseline walking speed, a threshold chosen as one that is likely to indicate a true change in walking speed over background fluctuations. A larger proportion of the Fampridine-SR treated subjects showed such improvements, but this difference was not statistically significant, given the sample size and placebo response rate.

Given the often large variations in function experienced by people with MS, it is difficult for the subject or a trained observer to separate a treatment-related improvement from a disease-related improvement without the element of consistency over time. Consistency of benefit might therefore be expected to be a more selective measure of true treatment effect than magnitude of change. Based on this rationale, the responses of the individual subjects in the MS-F202 trial were examined for the degree to which their walking speed showed improvement during the double-blind treatment period and returned towards pre-treatment values after they were taken off drug, at follow-up. This subject-by-subject examination yielded a subgroup of subjects whose pattern of walking speed over time appeared to be consistent with a drug response. This led to the analysis illustrated in Figure 1. This compares the placebo and Fampridine-SR treated groups with respect to the number of visits during the double-blind treatment period in which walking speed on the TW25 was faster than the maximum speed out of all five of the non-treatment visits (four visits prior to randomization and one follow-up visit after the drug treatment period).

The placebo-treated group showed a clear pattern of exponential decline in numbers of subjects with higher numbers of "positive" visits. This is what would be expected from a random process of variability. In contrast, the pattern of response in the Fampridine-SR treated group strongly diverged from this distribution; much larger numbers of Fampridine-SR treated subjects showed three or four visits with higher walking speeds than the maximum speed of all five non-treatment visits and less than half of the expected proportion had no visits with higher speeds. These results indicate that there was a sub-population of subjects in the Fampridine-SR treated group that experienced a consistent increase in walking speed related to treatment.

This analysis suggests that a relatively highly selective criterion for a likely treatment responder would be: a subject with a faster walking speed for at least three (i.e., three or four) of the four visits during the double blind treatment period compared to the maximum value for all five of the non-treatment visits. The four visits before initiation of double-blind treatment provide an initial baseline against which to measure the consistency of response during the four treatment visits. The inclusion of the follow-up visit as an additional component of the comparison was found valuable primarily in excluding those subjects who did not show the expected loss of improvement after coming off the drug. These are likely to be subjects who happened by chance to have improved in their MS symptoms around the time of treatment initiation, but whose improvement did not reverse on drug discontinuation because it was actually unrelated to drug. Thus, incorporating the follow-up visit as part of the criterion may help to exclude false positives, if the TW25 speed remains high at follow-up.

As described in Example 5 in *Appendix A,* this responder criterion was met by 8.5%, 35.3%, 36.0%, and 38.6% of the subjects in the placebo, 10 mg, 15 mg, and 20 mg b.i.d. treatment groups, respectively, showing a highly significant and consistent difference between placebo and drug treatment groups. Given that there was little difference in responsiveness between the three doses examined, more detailed analyses were performed comparing the pooled Fampridine-SR treated groups against the placebo-treated group. The full results of this analysis for study are described in the following sections. These show that the responder group so identified experienced a >25% average increase in walking speed over the treatment period and that this increase did not diminish across the treatment period. The responder group also showed an increase in Subject Global Impression score and an improvement in score on the MSWS-12. Thus, when utilizing the inventive process and software, it became possible to identify responders experienced clinically meaningful improvements in their MS symptoms, and treatment with fampridine significantly increased the chances of such a response. In doing so, a baseline was established showing comparability among the responder analysis groups, and then analyses were performed on the baseline demographic variables, key neurological characteristics and the relevant efficacy variables at baseline. In general, the responder analysis groups were comparable for all demographic and baseline characteristics variables, with certain exceptions.

Having demonstrated the clinical meaningfulness of consistently improved walking speeds during the double-blind period as a criterion for responsiveness, the question of the magnitude of benefit becomes of interest. The observed differences between the fampridine responders and the placebo group for the functional variables in this study are exactly what we would expect to see in the functional variables in an enrichment study where after a run-in period, only fampridine responders are entered, followed by a washout and randomization to either placebo or fampridine. The fampridine non-responders, although providing no relevant efficacy information, do provide safety information regarding those individuals who are treated with fampridine but show no apparent clinical benefit. As such, responder analyses of these groups were performed.

In one further exemplary aspect, a method of selecting individuals based on responsiveness to a treatment is derived from executing a range disparity distribution and applying it in a clinical trial setting. In this spect, a novel "range disparity"

(RD) distribution (RDD) is used to compute the probability that a given number of items (such as patients) in one set fall outside the range, on a give measure, of all the items (patients) in another set. Application of this distribution to evaluation of data from a real clinical trial is described and demonstrates an efficient new form of response analysis. As will be appreciated by those skilled in the art, many additional applications of the range distribution in clinical and other settings may be developed.

The exemplary particulars of the fundamental principle behind a range distribution may be described in the following rudimentary fashion. Suppose that there are three urns; call them X, Y, and Z. Suppose urn Z contains 10 straws of slightly different lengths. A referee selects five straws, places them into urn X and places the remaining five straws into urn Y. What is the probability distribution that a given number of straws in urn Y are longer than the longest straw in urn X?

• The probability is 5 out of 10 for urn X to provide the longest straw. Similarly, there is a 5/10 chance that urn Y will have no straws larger than the largest straw in urn X.

• For urn Y to have exactly one straw larger than the largest straw in urn X:

  ◦ urn Y must first have the largest straw (a 5/10 chance);
  ◦ the 5 straws in urn X must be the largest among the remaining 9 straws (a 5/9 chance).

So the probability for urn Y to have exactly one straw larger than the largest straw in urn X is x 5/10 x 5/9.

• For urn Y to have exactly two straws larger than the largest straw in urn X, urn Y must first have:

  ◦ the largest straw to begin with (a 5/10 chance);

  ◦ the second largest straw among the remaining 9 (a 4/9 chance);

  ◦ the 5 straws in urn X must be largest among the remaining 8 straws (a 5/8 chance).

So the probability for urn Y to have exactly two straws larger than the largest straw in urn X is x 5/10 x 4/9 x 5/8 = 5/10 x 5/9 x 4/8

• Continuing this logic, if we let the random variable T represent the number of straws in urn Y that are larger than the largest straw in urn X the we obtain the following distribution:

| t | [P (T=t)] | [P (T=t)] x 100% |
|---|---|---|
| 0 | 5/10 | 50.00% |
| 1 | 5/10 x 5/9 | 27.78% |
| 2 | 5/10 x 5/9 x 4/8 | 13.89% |
| 3 | 5/10 x 5/9 x 4/8 x 3/7 | 5.95% |
| 4 | 5/10 x 5/9 x 4/8 x3/7 x 2/6 | 1.98% |
| 5 | 5/10 x 5/9 x 4/8 x3/7 x 2/6 x 1/5 | 0.40% |
| Note that the probability of any event (i.e. T $\in$ {0, 1, 2, 3, 4, 5}) is 1. | | |

As another example, suppose the urn Z has 8-straws of different length, 5 of which are placed into urn X and 3 into urn Y. What is the probability distribution that a given number of straws in urn Y are longer than the longest straw in urn X? By the equivalent logic described above, we obtain the following distribution :

| t | [P (T=t)] | [P (T=t)] x 100% |
|---|---|---|
| 0 | 5/8 | 62.50% |
| 1 | 5/8 x 3/7 | 26.79% |
| 2 | 5/8 x 3/7 x 2/6 | 8.93% |
| 3 | 5/8 x 3/7 x 2/6 x 1/5 | 1.79% |
| Note that the probability of any event (i.e. T $\in$ {0, 1, 2, 3}) is 1. | | |

Using several combinations of straws in urn X and urn Y, the problem can be generalized for urn X to contain S-straws and urn Y to contain T-straws. This leads to the following definition.

**Definition 1:** Let N represent the set of positive integers. A random variable Y has the distribution, which we will call the Range Disparity Distribution (RDD) when (for S and $T \in \mathbb{N}$ and Y $\in$ 0 $\cap$ N such that $0 \le Y \le T$)

$$P(Y = y) = \begin{cases} \dfrac{S}{S+T}, & y = 0 \\[2ex] \dfrac{S}{S+T} \times \prod_{k=1}^{y} \dfrac{T-k+1}{S+T-k}, & otherwise \end{cases} \qquad (1)$$

This leads to the corresponding cumulative distribution function F(y):

$$F(y) = P(Y \leq y) = \begin{cases} 0, & y < 0 \\[2ex] \dfrac{S}{S+T}, & y = 0 \\[2ex] \sum_{j=0}^{y} P(Y = j), & otherwise \end{cases} \qquad (2)$$

While the preceding discussion supplies the probability distribution for the number of cases where items from X exceed the range of the items from Y, the same considerations will cover the opposite case: the number of cases where the items from X fall below the range of the items from Y.

This distribution has numerous potential applications: for example, in a clinical trial where measurements of a particular aspect of disease show essentially random variation with time. In such a case, we may be constrained (for example by clinic visit schedules) to obtain only a small sample of measurements from each patient over the course of a baseline period and a small sample of measurements over a treatment period. The RDD provides a simple and effective way to identify individuals who show an unexpected range-shift in either the positive or negative direction, indicating either a consistent benefit or a consistent worsening that is temporally associated with the treatment. In addition to making between group comparisons, we can compare the distribution of changes in the placebo group to the expected RDD to identify and measure any temporal changes due to factors such as the placebo effects and natural disease progression or remission.

Consistency of benefit from treatment would be expected to be a more effective measure of response (i.e. of causality) than simply examining the magnitude of change between the average baseline visit and the average treatment visit. This is because a meaningful, consistent benefit may be small in magnitude and a large random deviation, occurring during any individual measurement, can have a substantial but ultimately meaningless effect on the average value across a small number of sample measurements.

**Example 1 - Theoretical basis:** Assuming a clinical trial such that for each patient there are S off-drug measurements of a particular affected function and T on-drug measurements. Let Y represent the number of on-drug measurements that are better (e.g. more normal) than the best off-drug measurement. Assume Y follows the range disparity (RD) distribution. For example, if there are S=5 off-drug visits and T=5 on-drug visits then the probability distribution of Y, is:

| y | [P (Y=y)] x 100% |
|---|---|
| 0 | 50.00% |
| 1 | 27.78% |
| 2 | 13.89% |
| 3 | 5.95% |
| 4 | 1.98% |
| 5 | 0.40% |

This distribution implies that, if the active treatment has no effect we would expect the proportion of patients who experience a consistent improvement, reflected by 4 or 5 on-drug measurement better than the best off-drug measurement, to be about 2.5%. The null hypothesis that the groups are equal with regard to the proportion of subjects with consistent improvement can be tested using a standard test such as Fisher's exact test, a chi-square test, or for stratified samples (e.g., by study center) the Cochran-Mantel-Haenszel test. Significant departures from this expected frequency in the active treatment group, but not the placebo group, would lead us to conclude that the treatment and placebo groups are different. Significant differences between all three distributions (active treatment, placebo, and expected RD), would indicate a treatment effect superimposed on a temporal change due to other factors.

Hence, the identification of a consistent response as represented by 4 or 5 of the on-drug measurements as better than the best off-drug measurement provides a particularly clear criterion for a responder analysis. A traditional responder analysis would establish an arbitrary level of average change (e.g. 10%, 20%) above which a trial subject would qualify as a responder. Generally, there is no clear clinical or statistical justification for such a criterion and no a priori method for its estimation. On the other hand, a criterion of consistency based on the RDD can be clinically meaningful (being based on consist relationship to treatment over time), statistically appropriate (based on a threshold of statistical probability, here approximately 2.5% for a one-sided criterion.) and it can be calculated a priori, given the trial design.

Below is a brief outline of a general approach to determine appropriate parameters for a responder criterion based on the concept of consistency across measurements. A general approach to determine an appropriate response criterion for a clinical trial might be derived as follows:

Let,

$X_{is}$ (i=1,2,, ..., I and s=1,2, ...S) represent the $s^{th}$ off-drug measurement for patient i .
$Y_{it}$ (i=1,2,, ..., I and t=1,2, ...T) represent the $t^{th}$ on-drug measurement for patient i .

Assumptions:

- Each X and Y measurement addresses the same outcome variable: Z (we use X and Y to differentiate measurements during different time-periods: off-drug and on-drug).
- Initially assume no treatment effect and that there is no longitudinal effect on the outcome measure. That is to say that over time, there is at best, negligible within-patient correlation. If such an effect exists, it will become apparent in the analysis itself.

Set up a consistency criterion C which is a relation ($\rho$) between the off-drug measurements and each on-drug measurement such that:

$$C_{it} = \begin{cases} 1 \ if \ f(X_{is}) \ \rho \ Y_{it} \\ 0 \qquad otherwise \end{cases}$$

and compute the number of on-drug visits that fulfill the criterion $C_i = \sum_{i=1}^{T} C_{it}$ . choose a value $\lambda \leq T$ such that a responder criterion is defined as:

$$C_{iR} = \begin{cases} 1 \qquad if \ C_i \geq \lambda \\ 0 \qquad otherwise \end{cases}$$

For clinical trials, a good rule of thumb is to choose $\lambda$ such that the theoretical responder rate is no larger than 5%, i.e.:

$$0 \leq [P(C_{iR}=1] \leq 0.05$$

**Example 2 - Practical experience:** The following is based on data from a clinical trial that examined the effects of a novel treatment in improving walking speed in patients diagnosed with a chronic disease and was designed with 5 off-drug and 4 on-drug assessments of walking speed. Subjects were randomized to receive active drug or placebo in a 3:1 ratio. For a given patient, if we let Y represent the number of on-drug measured walking speeds that are faster than the fastest off-drug walking speed and assume Y follows the RDD we have:

Table 1. Table showing the theoretical distribution of on-drug visits with faster walking speeds than the fastest off-drug walking speed using the RDD.

| y | $[P (Y=y)]$ x 100% |
|---|---|
| 0 | 55.56% |
| 1 | 27.78% |
| 2 | 11.90% |
| 3 | 3.97% |
| 4 | 0.79% |

Applying the general approach to determine an appropriate response criterion, response to treatment was defined as a faster walking speed in at least 3 of the 4 on-drug visits compared to the fastest speed measured during the 5 off-drug visits. There were 205 intent-to-treat patients included in the primary efficacy analysis (47 placebo and 158 active treatment). Table 2 below summarizes the key study result.

Table 2. Table showing the percentage of responders, selected for consistent improvement in walking speed in the placebo-treated and active-drug treated groups. P-value calculated from the Cochran-Mantel-Haenszel test, controlling for study center.

| | Placebo (N=47) | Drug (N=158) |
|---|---|---|
| Percentage of Responders | 8.5% | 36.7% |
| p-value | < 0.001 | |

As can be seen, the placebo responder rate (8.5%) was very close to the theoretical responder rate of about 5%. Indeed, when we examine the frequency distribution for the placebo-treated group in Graph A, below, we see that the observed distribution of better on-drug measurements was similar to that expected from the RDD, thereby suggesting negligible temporal or placebo effects in this trial. On the other hand, the distribution of measurements in the actively treated group was significantly different from both the placebo and the theoretical distributions. In particular, there were large differences in the proportion of subjects showing no measurements faster than the fastest off-drug measurement and showing 3 or 4 faster visits. This indicates that active treatment but not placebo treatment is associated with more consistent improvement than would be expected from the RDD, and that our selection of the response criterion based on statistical probability is reasonable in practice.

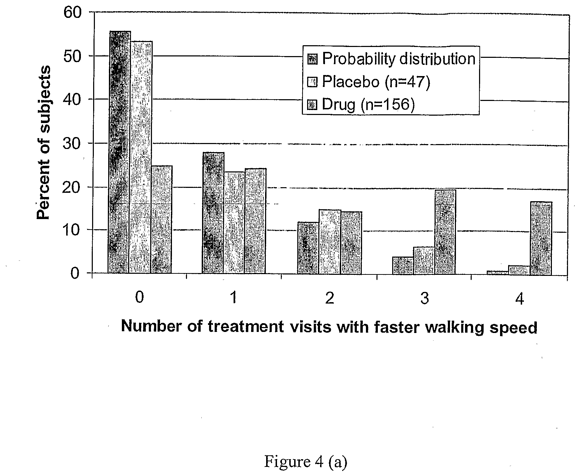

Figure 4 (a): Histogram to show the proportion of subjects in the two treatment groups who experienced a given number of walking assessments during treatment that were faster than the fastest speed measured during the off-treatment period. These distributions are compared to the expected RD probability distribution.

The utility of this form of response analysis is shown both by the differentiation of treatment and control groups and by the ability to show that, in the absence of active treatment, there was no significant independent shift in the placebo group that would indicate treatment-independent changes related to time or to placebo-effects.

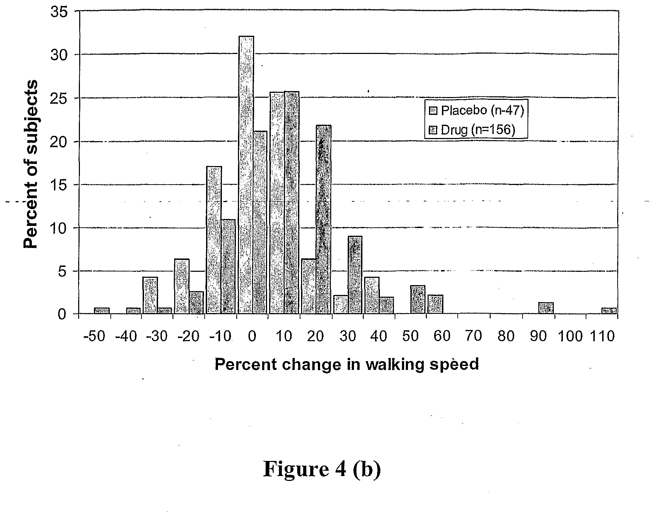

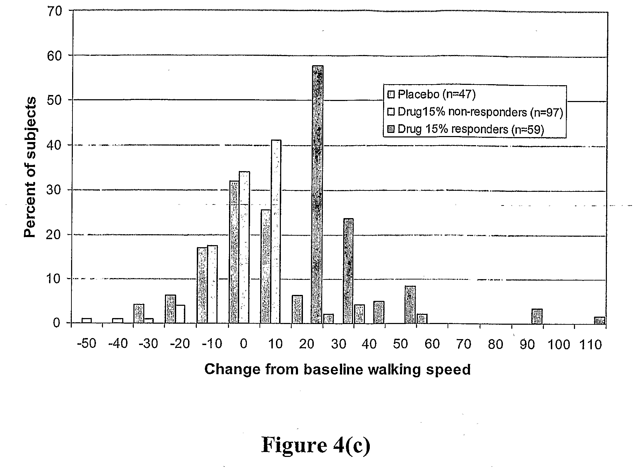

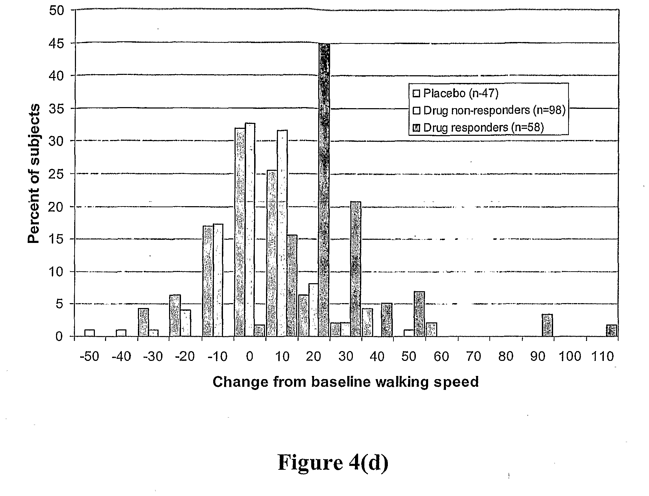

The application of this criterion for "consistent response analysis" allows very efficient sampling. This is shown by comparing three forms of analyzing the data from this study in Figures 4 (b)-(d). Simply by examining the distribution of mean changes between baseline and treatment periods in a traditional quantitative comparison (in Figure 4(c)) we see a trend for improvement in the drug-treated group, and we would expect such a difference to be resolved with statistical significance in a much larger study). However, in this study the difference was not significant by ANOVA. A traditional response analysis, setting a threshold of 15% improvement (Figure 4 (c)) shows again a clear favorable trend, with many more "responders" in the active group, but this is not statistically significant with the sample size available (using the Cochran-Mantel Haenszel test). Similar results are achieved with other arbitrary response thresholds, e.g. 10% or 20%. Applying the "consistent response" criterion, we see a highly statistically significant response with the available sample size (Figure 4(d), Table 2). Not only is the overall analysis rendered more sensitive by the consistent response criterion, but this approach, unlike the other two, also allows us: a) to show that the placebo response rate is not significantly different from that expected by random variability (Figure 4 (a), Table 2); and b) to know before the trial that the expected false-positive rate for the criterion is approximately 5% (Table 1) based on the RD probability distribution.

Figure 4(b) : Distribution of changes in average walking speed between the baseline, pre-treatment period and the double-blind treatment period for patients randomized to either placebo treatment or active treatment. Although there appears to be a difference in mean changes between treatment groups, this was not statistically significant based on ANOVA.

Figure 4 (c): Applying a traditional responder analysis to the data in Figure 4 (b), with a threshold for "response" set at >15% improvement. This shows that the distribution of changes among non-responders in the drug-treated group is very different from that of the placebo group, indicating the presence of significant numbers of false negatives among non-responders and false positives among responders. A total of 7 placebo-treated patients registered as responders, for a 14.8% false-positive rate in that group. The response rate in the drug-treated group was 44.2%. The drug-placebo response differential was therefore 29.4%, but this was not statistically significant. Clearly, the threshold chosen for the response definition will affect the numbers of responders in both treatment groups but would not change the arbitrary nature of the division between responders and non-responders.

Figure 4(d) : Applying the consistent (repeated measures) response analysis to the same data from Figure 4 (b) selects out a non-responder population that is close to the placebo-treated group in its distribution. Only 4 subjects in the placebo group registered as responders, for an 8.5% false positive rate. The response rate in the drug-treated group was 37.2%. The drug-placebo response differential was therefore 28.7%, similar to the 29.3% seen with the traditional responder analysis approach shown in Figure 4 (b). However, in this case there was a lower frequency of false positives in the placebo group, and the difference between treatment group response rates was statistically significant ($p < 0.001$, Table 1).

**Example 3 :** The application of this distribution is particularly powerful in the context of a repeated measures response analysis of the kind provided by Example 2. However, it may be useful in various simpler situations, for example, in a case of industrial product sampling. There might be a suspicion that plant A is producing items with a breaking strength that is lower than those from plant B. For destructive testing of items from the two plants we would likely want to minimize the sample size. If we sampled 5 out of 100 items from the next production run at each plant and determine that the breaking strength of more than 2 of these items from plant A falls below the range of the 5 tested from plant B we would have support for the suspicion regarding a difference between the plants. More specifically, we would know that there is less than a 2.5% chance, on the basis of random variability (Example 1), that 4 or 5 of the samples from A would fall below the failure range for those from plant B.

The range disparity distribution, therefore describes the expected behavior of two small samples from a common population. Specifically, it defines the probability that any given number of values in one sample from that population will fall outside the range of values in the other sample, in either the positive or negative direction. This distribution can be applied to novel forms of small-sample statistical analysis. An example of application to a repeated measures response analysis in a clinical trial is described. The definition of a consistent response, based on the sample range disparity distribution, improves the sensitivity as well as the statistical and clinical meaningfulness of such an analysis.

**Example 4:** In addition, the method, system and software of the present invention was utilized in the testing of Fampridine-SR on walking in people with multiple sclerosis (MS) during a Phase 3 trial, the results of which were announced on September 25, 2006. In particular, this Phase 3 clinical trial of Fampridine-SR on walking in people with multiple sclerosis (MS) was a confirmation of the pertinence of the inventive approach. In utilizing the method, system and software of the present invention, statistical significance was achieved on all three efficacy criteria defined in the Special Protocol Assessment (SPA) by the Food and Drug Administration (FDA). As a result of utilizing the inventive techniques, a significantly greater proportion of people taking Fampridine-SR had a consistent improvement in walking speed, the study's primary outcome, compared to people taking placebo (34.8 percent vs. 8.3 percent) as measured by the Timed 25-Foot Walk (p less than 0.001). In addition, the effect was maintained in this study throughout the 14-week treatment period (p less than 0.001) and there was a statistically significant improvement in the 12-Item MS Walking Scale (MSWS-12) for walking responders vs. non-responders (p less than 0.001). The average increase in walking speed over the treatment period compared to baseline was 25.2 percent for the drug-responder group vs. 4.7 percent for the placebo group. Increased response rate on the Timed 25-Foot Walk was seen across all four major types of MS. In addition, statistically significant increases in leg strength were seen in both the Fampridine-SR Timed Walk responders (p less than 0.001.) and the Fampridine-SR Timed Walk non-responders (p=0.046) compared to placebo.

Although the present invention has been described in considerable detail with reference to certain preferred embodiments thereof, other versions are possible. Therefore the scope of the appended claims should not be limited to the description and the preferred versions contain within this specification.

### Appendix A

The below text is reproduced merely for illustrative purposes, was excerpted from US Pat. Application No 11/102,559, the entirety of which is hereby incorporated by reference.

**EXAMPLE 5**

This example provides an embodiment of a method of treating subjects with a sustained release fampridine formulation and a responder analysis of the present invention. This was a Phase 2, double-blind, placebo-controlled, parallel group, 20-week treatment study in 206 subjects diagnosed with Multiple Sclerosis. This study was designed to investigate the safety and efficacy of three dose levels of Fampridine-SR, 10 mg b.i.d., 15 mg b.i.d., and 20 mg b.i.d. in subjects with clinically definite MS. The primary efficacy endpoint was an increase, relative to baseline, in walking speed, on the Timed 25 Foot Walk. Secondary efficacy measurements included lower extremity manual muscle testing in four groups of lower extremity muscles (hip flexors, knee flexors, knee extensors, and ankle dorsiflexors); the 9-Hole Peg Test and Paced Auditory Serial Addition Test (PASAT 3"); the Ashworth score for spasticity; Spasm Frequency/Severity scores; as well as a Clinician's (CGI) and Subject's (SGI) Global Impressions, a Subject's Global Impression (SGI), the Multiple Sclerosis Quality of Life Inventory (MSQLI) and the 12-Item MS Walking Scale (MSWS-12).

At the first visit (Visit 0) subjects were to enter into a two-week single-blind placebo run-in period for the purpose of establishing baseline levels of function. At Visit 2 subjects were to be randomized to one of four treatment groups (Placebo or Fampridine-SR 10 mg, 15 mg, 20 mg) and begin two weeks of double-blind dose-escalation in the active drug treatment groups (B, C and D). Group A were to receive placebo throughout the study. Subjects in the 10 mg (Group B) arm of the study took a dose of 10 mg approximately every 12 hours during both weeks of the escalation phase. The 15 mg (Group C) and 20 mg (Group D) dose subjects took a dose of 10 mg approximately every 12 hours during the first week of the escalation phase and titrated up to 15 mg b.i.d. in the second week. Subjects were to be instructed to adhere to an "every 12 hour" dosing schedule. Each subject was advised to take the medication at approximately the same time each day throughout the study; however, different subjects were on differing medication schedules (e.g., 7 AM and 7 PM; or 9 AM and 9 PM). After two weeks, the subjects were to return to the clinic at Visit 3 for the start of the stable dose treatment period. The first dose of the double-blind treatment phase at the final target dose (placebo b.i.d. for the Group A, 10 mg b.i.d. for Group B, 15 mg b.i.d. for Group C, and 20 mg b.i.d. for Group D) was taken in the evening following Study Visit 4. Subjects were to be assessed five times during the 12-week treatment period. Following the 12-week treatment phase there was to be a one-week down titration starting at Visit 9. During this down-titration period, group B was to remain stable at 10 mg b.i.d. and Group C was to be titrated to 10 mg b.i.d., while group D was to have a change in the level of dose during the week (15 mg b.i.d. for the first three days and 10 mg b.i.d. for the last four days). At the end of the down titration period at Visit 10, subjects were to enter a two-week washout period where they did not receive any study medication. The last visit (Visit 11) was to be scheduled two weeks after the last dosing day (end of the downward titration). Plasma samples were collected at each study site visit other than Study Visit 0.

The primary measure of efficacy was improvement in average walking speed, relative to the baseline period (placebo run-in), using the Timed 25 Foot Walk from the Multiple Sclerosis Functional Composite Score (MSFC). This is a quantitative measure of lower extremity function. Subjects were instructed to use whatever ambulation aids they normally use and to walk as quickly as they could from one end to the other end of a clearly marked 25-foot course. Other efficacy measures included the LEMMT, to estimate muscle strength bilaterally in four groups of muscles: hip flexors, knee flexors, knee extensors, and ankle dorsiflexors. The test was performed at the Screening Visit and at Study Visits 1, 2, 4, 7, 8, 9 and 11. The strength of each muscle group was rated on the modified BMRC scale: 5 = Normal muscle strength; 4.5= Voluntary movement against major resistance applied by the examiner, but not normal; 4= Voluntary movement against moderate resistance applied by the examiner; 3.5= Voluntary movement against mild resistance applied by the examiner; 3= Voluntary movement against gravity but not resistance; 2= Voluntary movement present but not able to overcome gravity; 1= Visible or palpable contraction of muscle but without limb movement; and 0= Absence of any voluntary contraction. Spasticity in each subject was assessed using the Ashworth Spasticity Score. The Ashworth Spasticity Exam was performed and recorded at the Screening Visit and at Study Visits 1, 2, 4, 7, 8, 9 and 11.

Protocol Specified Responder Analysis. To supplement the primary analysis, a categorical "responder" analysis was also conducted. Successful response was defined for each subject as improvement in walking speed (percent change from baseline) of at least 20%. Subjects who dropped out prior to the stable dose period were considered non-responders. The proportions of protocol specified responders were compared among treatment groups using the Cochran-Mantel-Haenszel test, controlling for center.

Post hoc analysis of this study suggested that a relatively highly selective criterion for a likely treatment responder would be a subject with a faster walking speed for at least three visits during the double blind treatment period as compared to the maximum value among a set of five non-treatment visits (four before treatment and one after discontinuation of treatment). The four visits before initiation of double-blind treatment provided an initial baseline against which to measure the consistency of response during the four double-blind treatment visits. The inclusion of the follow-up visit as an additional component of the comparison was useful primarily in excluding those subjects who may be false positives, i.e., did not show the expected loss of improvement after coming off the drug. Treatment differences in the proportion of theses post hoc responders were analyzed using the Cochran-Mantel-Haenszel (CMH) test, controlling for center.

To validate the clinical meaningfulness of the post hoc responder variable, (post hoc) responders were compared against the (post hoc) non-responders, on the subjective variables: (i) Change from baseline in MSWS-12 over the double-blind; (ii) SGI over the double-blind; and (iii) Change from baseline in the CGI over the double-blind; to determine if subjects with consistently improved walking speeds during the double-blind could perceive improvement relative to those subjects who did not have consistently improved walking speeds. For the subjective variables, differences between responder status classification (responder or non-responder) were compared using an ANOVA model with effects for responder status and center.

Results. A total of 206 subjects were randomized into the study: 47 were assigned to placebo, 52 to 10 mg bid Fampridine-SR (10 mg bid), 50 to 15 mg bid Fampridine-SR (15 mg bid), and 57 to 20 mg bid Fampridine-SR (20 mg bid). The disposition of subjects is presented in Table 5 below.

**Table 5 Summary of subject disposition (all randomized population)**

|  | Treatment Group: N (%) | | | | |
|---|---|---|---|---|---|
|  | Placebo | 10 mg bid | 15m g bid | 20 mg bid | Total |
| **Subjects Randomized** | 47 | 52 | 50 | 57 | 206 |
| **Took at Least One Dose (Included in Safety Analysis)** | 47 (100%) | 52 (100%) | 50 (100%) | 57 (100%) | 206 (100%) |
| **ITT Population** | 47 (100%) | 51 (98.1%) | 50 (100%) | 57 (100%) | 205 (99.5%) |
| **Discontinued Subjects** | 2 (4.3%) | 2 (3.8%) | 1 (2.0%) | 6 (10.5%) | 11 (5.3%) |
| Note: Percentages are based on the number of randomized subjects. | | | | | |

All 206 randomized subjects took at least one dose of study medication and were included in the safety population. One subject (subject# 010/07 10 mg bid group) was excluded from the ITT population (lost to follow-up after 8 days of placebo run-in). A total of 11 subjects discontinued from the study.

The population consisted of 63.6% females and 36.4% males. The majority of the subjects were Caucasian (92.2%), followed by Black (4.9%), Hispanic (1.5%), those classified as 'Other' (1.0%), and Asian/Pacific Islander (0.5%). The mean age, weight, and height of the subjects were 49.8 years (range: 28-69 years), 74.44 kilograms (range: 41.4-145.5 kilograms), and 168.84 centimeters (range: 137.2-200.7 centimeters), respectively. Most of the subjects (52.4%) had a diagnosis type of secondary progressive with about equal amounts of relapsing remitting (22.8%) and primary progressive (24.8%) subjects. The mean duration of disease was 12.00 years (range: 0.1-37.5 years) while the mean Expanded Disability Status Scale (EDSS) at screening was 5.77 units (range: 2.5-6.5 units). The treatment groups were comparable with respect to all baseline demographic and disease characteristic variables.

Results for the key efficacy variables at baseline for the ITT population are further summarized in Table 6 below.

**Table 6 Summary of key efficacy variables at baseline (ITT population)**

| Parameter | Treatment Group: Mean (SD) | | | | |
|---|---|---|---|---|---|
|  | placebo N=47 | 10 mg bid N=51 | 15mg bid N=50 | 20 mg bid N=57 | Trea tment. p-value |
| **Walking Speed (ft/sec)** | 1.87 (0.902) | 1.94 (0.874) | 1.99 (0.877) | 2.04 (0.811) | 0.75 2 |
| **LEMMT** | 4.05 (0.690) | 3.98 (0.661) | 4.00 (0.737) | 3.98 (0.634) | 0.96 4 |
| **SGI** | 4.38 (0.795) | 4.32 (0.999)* | 4.56 (1.110) | 4.25 (0.969) | 0.41 3 |
| **MSWS-12** | 75.71 (16.566) | 76.31 (16.186) | 74.60 (17.671) | 76.83 (18.124) | 0.92 3 |
| **\*: One subject did not have a baseline value.** | | | | | |

With respect to the 205 subjects in the ITT population, mean values for baseline walking speed, LEEMT, SGI, and MSWS-12 were approximately 2 feet per second, 4 units, 4.5 units, and 76 units, respectively. The treatment groups were comparable with respect to these variables as well as all the other efficacy variables at baseline.

Descriptive statistics for the average walking speed (ft/sec) by study day based on the Timed 25-Foot Walk are presented in Table 7 and Figure 2. The timed 25 foot walk showed a trend toward increased speed during the stable dose period for all three dose groups, though the average improvement declined during the treatment period.

**Table 7 Average walking speeds (ft/sec) by study day (observed cases, ITT population)**

| Summary Statistics Over Time | | | | | | | |
|---|---|---|---|---|---|---|---|
| | | Study day | | | | | |
| Treatment | | base | titration | 1st stbl | 2nd stbl | 3rd stbl | follow-up |
| placebo | Mean | 1.87 | 1.89 | 1.90 | 1.89 | 1.89 ' | 1.86 |
| | (SD) | (0.902) | (0.876) | (0.908) | (0.891) | (0.914) | (0.933) |
| | N# | 47 | 47 | 46 | 46 | 45 | 45 |
| 10mg bid | Mean | 1.94 | 2.20 | 2.09 | 2.12 | 2.00 | 1.88 |
| | (SD) | (0.874) | (0.979) | (0.955) | (1.043) | (1.016) | (0.970) |
| | N | 51 | 51 | 51 | 51 | 50 | 48 |
| 15mg bid | Mean | 1.99 | 2.25 | 2.16 | 2.14 | 2.18 | 1.83 |
| | (SD) | (0.877) | (0.995) | (0.986) | (0.957) | (0.932) | (0.952) |
| | N | 50 | 49 | 49 | 48 | 48 | 47 |
| 20mg bid | Mean | 2.04 | 2.26 | 2.22 | 2.19 | 2.04 | 1.83 |
| | (SD) | (0.811) | (0.936) | (0.893) | (0.936) | (0.996) | (0.822) |
| | N | 57 | 55 | 52 | 51 | 49 | 55 |
| **#: The treatment sample sizes presented in the figure legend represent the number of ITT subjects. Sample sizes at individual time points may be smaller than those in the ITT population due to dropouts or missed assessments.** | | | | | | | |

During double-blind treatment, all the Fampridine-SR groups exhibited mean walking speeds between 2.00 and 2.26 feet per second, while the mean value in the placebo group was consistently about 1.90 feet per second. It should be noted that, at the third stable-dose visit, both the 10 mg bid and 20 mg bid group means dropped-off from what would be expected under the assumption that treatment benefit is consistent over time. This may or may not have been due to chance; further studies should provide additional evidence for either case. After double-blind medication was discontinued, all the treatment groups converged to approximately the same mean value at follow-up.

Results for the primary efficacy variable (percent change in average walking speed during the 12-week stable dose period relative to baseline based on the 25-foot walk) are summarized in Figure 3. The timed 25 foot walk showed a trend toward increased speed during the stable dose period for all three dose groups, though the average improvement declined during the treatment period, as shown in Figure 3. The mean percent changes in average walking speed during the 12-week stable dose period (based on adjusted geometric mean change of the log-transformed walking speeds) were 2.5%, 5.5%, 8.4%, and 5.8% for the placebo, 10 mg bid, 15 mg bid, and 20 mg bid groups, respectively. There were no statistical differences between any Fampridine-SR groups and the placebo group.

Results for the protocol specified responder analysis (subjects with average changes in walking speed during the 12 weeks of stable double-blind treatment of at least 20%) are summarized in Figure 4. The percentages of subjects with average changes in walking speed during the 12-week stable dose period of at least 20% (pre-defined responders) were 12.8%, 23.5%, 26.5%, and 16.1% for the placebo, 10 mg bid, 15 mg bid, and 20 mg bid groups, respectively. There were no statistically significant differences between any of the Fampridine-SR groups and the placebo group.

Descriptive statistics for the average overall Lower Extremity Manual Muscle Testing (LEMMT) by study day are presented in Table 8 and in Figure 5.

**Table 8. Average overall LEMMT by Study Day**

| Summary Statistics Over Time | | | | | | | |
|---|---|---|---|---|---|---|---|
| | | Study day | | | | | |
| Treatment | | base | titration | 1st stbl | 2nd stbl | 3rd stbl | follow-up |
| placebo | Mean | 4.05 | 4.00 | 4.02 | 4.03 | 4.00 | 4.02 |
| | (SD) | (0.690) | (0.705) | (0.687) | (0.696) | (0.679) | (0.738) |
| | N# | 47 | 46 | 46 | 46 | 45 | 45 |
| 10mg bid | Mean | 3.98 | 4.09 | 4.06 | 4.09 | 4.07 | 3.89 |
| | (SD) | (0.661) | (0.641) | (0.650) | (0.685) | (0.642) | (0.631) |
| | N | 51 | 50 | 51 | 51 | 50 | 49 |
| 15mg bid | Mean | 4.00 | 4.16 | 4.11 | 4.09 | 4.17 | 4.08 |
| | (SD) | (0.737) | (0.653) | (0.645) | (0.659) | (0.618) | (0.674) |
| | N | 50 | 49 | 49 | 49 | 49 | 46 |
| 20mg bid | Mean | 3.98 | 4.08 | 4.03 | 3.98 | 4.07 | 3.92 |
| | (SD) | (0.634) | (0.639) | (0.659) | (0.714) | (0.649) | (0.650) |
| | N | 57 | 54 | 52 | 52 | 48 | 55 |
| **#: The treatment sample sizes presented at individual time points may be smaller than those in the ITT population due to dropouts or missed assessments.** | | | | | | | |

During double-blind treatment, all the Fampridine-SR groups exhibited a numerical pattern of larger mean LEMMT scores than placebo (except the 20 mg bid group at the 2nd stable dose visit). After double-blind medication was discontinued, with the exception of the 15 mg bid group, all the group means were lower than they were at baseline.

Results for the average change in LEMMT during the 12-week stable dose period relative to baseline are summarized in Figure 6. The mean changes in overall LEMMT during the 12-week stable dose period were -0.05 units, 0.10 units, 0.13 units, and 0.05 units for the placebo, 10 mg bid, 15 mg bid, and 20 mg bid groups, respectively. Improvements in LEMMT were significantly greater in the 10 mg bid and 15 mg bid groups compared to the placebo group; there was no significant difference between the 20 mg bid group and the placebo group.

No statistically significant differences were detected among treatment group based on any of the other secondary efficacy variables, as shown in Table 9.

**Table 9. Changes from baseline during the 12-week stable dose period in selected secondary efficacy variables (observed cases, ITT population)**

| Parameter | Treatment Group | | | |
|---|---|---|---|---|
| | placebo N=47 | 10 mg bid N=51 | 15mg bid N=50 | 20 mg bid N=57 |
| Ashworth Score<br>    N<br>    Mean (SD)<br>    p-value (each dose vs. placebo) | 46<br>-0.11 (0.377) | 51<br>-0.04 (0.449)<br>0.802 | 49<br>-0.06 (0.375)<br>0.826 | 53<br>0.02 (0.466)<br>0.275 |
| CGI<br><br>    Mean (SD)<br>    p-value (each dose vs. placebo) | 45<br><br>0.0 (0.66) | 50<br><br>-0.2 (0.72)<br>0.772 | 49<br><br>-0.1 (0.85)<br>0.997 | 52<br><br>0.0 (0.78)<br>0.996 |
| SGI<br><br> | 46 | 50 | 49 | 53 |

(continued)

| | | | | |
|---|---|---|---|---|
| SGI | | | | |
| Mean (SD) | -0.2 (0.96) | 0.0 (1.27) | -0.1 (1.11) | -0.1 (0.86) |
| p-value (each dose vs. placebo) | | 0.704 | 0.953 | 0.968 |
| PASAT | | | | |
| | | 46 | 51 | 49 | 53 |
| Mean (SD) | 2.17 (4.016) | 2.13 (3.394) | 0.90 (3.274) | 0.65 (4.590) |
| p-value (each dose vs. placebo) | | >0.999 | 0.306 | 0.218 |
| MSFC | | | | |
| | 46 | 51 | 49 | 52 |
| Mean (SD) | 0.08 (0.205) | 0.10 (0.310) | 0.90 (0.224) | 0.06 (0.194) |
| p-value (each dose vs. placebo) | | 0.977 | >0.999 | 0.968 |
| MSWS-12 | | | | |
| | 46 | 51 | 49 | 52 |
| Mean (SD) | -3.56 (14.548) | -5.53 (16.154) | -7.32 (16.295) | -5.76 (15.296) |
| p-value (each dose vs. placebo) | | 0.718 | 0.445 | 0.617 |

**Note: The treatment sample sizes presented in the treatment heading represent the number of ITT subjects. Sample sizes for individual variables may be smaller due to dropouts or missed assessments.**
Note: For each variable, the p-values (versus placebo) are Dunnett-adjusted.

While pre-planned analyses of the primary efficacy endpoint provided insufficient evidence of treatment benefits for any of the Fampridine-SR doses, subsequent analysis revealed the existence of a subset of subjects who responded to the drug with clinical meaningfulness. These subjects exhibited walking speeds while on drug that were consistently better than the fastest walking speeds measured when the subjects were not taking active drug.

The post hoc responder rates based on consistency of improved walking speeds were significantly higher in all three active dose groups (35, 36 and 39%) compared to placebo (9%; p<0.006 for each dose group, adjusting for multiple comparisons) as shown in Figure 7.

Given that there was little difference in responsiveness between the three doses examined, more detailed analyses were performed comparing the pooled Fampridine-SR treated groups against the placebo-treated group. Figure 8 summarizes, for the placebo and the pooled Fampridine-SR group, the percentage of post hoc responders. The number of subjects who met he post hoc responder criterion in the pooled Fampridine-SR treated group was 58 (36.7%) compared to 4 (8.5%) in the placebo-treated group, and this difference was statistically significant (p<0.001).

To validate the clinical meaningfulness of the post hoc responder variable, the 62 responders (58 fampridine and 4 placebo) were compared against the 143 non-responders (100 fampridine and 43 placebo) on the subjective variables to determine if subjects with consistently improved walking speeds during the double-blind could perceived benefit relative to those subjects who did not have consistently improved walking speeds. The results are summarized in Figure 9 and indicate that consistency in walking speed had clinical meaningfulness for the subjects in this study since the responders had (over the double-blind period) significantly better changes from baseline in MSWS-12 and significantly better subjective global scores. In addition, the responders were rated marginally better than the non-responders by the clinicians during the double-blind. Thus, responders experienced clinically meaningful improvements in their MS symptoms, and treatment with fampridine significantly increased the chances of such a response.

To establish baseline comparability among the responder analysis groups, analyses were performed on the baseline demographic variables, key neurological characteristics and the relevant efficacy variables at baseline. In general, the responder analysis groups were comparable for all demographic and baseline characteristics variables.

Having demonstrated the clinical meaningfulness of consistently improved walking speeds during the double-blind as a criterion for responsiveness, the question of the magnitude of benefit becomes of interest. The observed differences between the fampridine responders and the placebo group for the functional variables in this study are exactly what we would expect to see in the functional variables in an enrichment study where after a run-in period, only fampridine responders are entered, followed by a washout and randomization to either placebo or fampridine. The fampridine non-responders, although providing no relevant efficacy information, do provide safety information regarding those individuals who are treated with fampridine but show no apparent clinical benefit. As such, responder analyses of these groups were performed.

With respect to magnitude of benefit, Figure 10 and Table 12 below summarizes the percent changes in walking speed at each double-blind visit by responder analysis grouping. The mean improvement for the fampridine responders during the double-blind across 14 weeks of treatment ranged from 24.6% to 29.0% compared to 1.7% to 3.7% for the placebo group; this was highly significant (p<0.001) at every visit. Although providing no relevant efficacy information, results for the fampridine non-responders are also illustrated and show that there was, and could be, some worsening in walking speeds after 12-weeks when a non-responder is treated with fampridine. The improvement was stable ($\pm$ 3%) across 14 weeks of treatment, and was associated with improvement in two global measures (Subject Global Impression and Multiple Sclerosis Walking Scale-12). The four placebo responders showed a 19% improvement in walking speed but there were too few subjects in this group for meaningful statistical comparison. Response status was not significantly related to baseline demographics, including type or severity of MS. Adverse events and safety measures were consistent with previous experience for this drug.

**Table 12. Summary of percent change in Walking Speed at each double-blind visit by responder analysis grouping.**

| | | | | | |
|---|---|---|---|---|---|
| Summary Statistics Over Time | | | | | |
| | | Study day | | | |
| Treatment | | titration | 1st stbl | 2nd stbl | 3rd stbl |
| Placebo | Mean | 1.7 | 2.6 | 1.8 | 3.7 |
| | (SEM) | (2.21) | (3.23) | (3.11) | (3.38) |
| | N# | 47 | 46 | 46 | 45 |
| Fampridine | Mean | 8.3 | 3.5 | -0.2 | -6.5 |
| Non-responders | (SEM) | (2.05) | (1.90) | (1.76) | (2.49) |
| | N | 97 | 94 | 93 | 89 |
| Fampridine | Mean | 27.4 | 24.6 | 29.0 | 27.3 |
| Responders | (SEM) | (2.43) | (2.44) | (4.31) | (3.52) |
| | N | 58 | 58 | 57 | 58 |
| FR vs. Placebo | p-value^ | <0.001 | <0.001 | <0.001 | <0.001 |
| FR vs. FNR | p-value^ | <0.001 | <0.001 | <0.001 | <0.001 |
| FNR vs. PBO | p-value^ | 0.080 | 0.884 | 0.497 | 0.022 |

ABBREVIATIONS: FR=Fampridine Responders; FNR=Fampridine Non-responders.
**: Significantly better than placebo and fampridine non-responders (p < 0.001 for each).
*: Significantly better than fampridine non-responders.
#: The treatment sample sizes presented at individual time points may be smaller than those in the ITT population due to dropouts or missed assessments.
#: The treatment sample sizes presented in the figure legend represent the number of ITT subjects. Sample sizes at individual time points may be smaller due to dropouts or missed assessments.
^: P-values from t-tests of the least-squares means using the mean square error via an ANOVA model with effects for responder analysis grouping and center.

Figure 11 and Table 13 summarize the changes in LEMMT at each double-blind visit by responder analysis grouping. The mean improvement for the fampridine responders during the double-blind ranged from 0.09 to 0.18 units compared to -0.04 units at each visit for the placebo group; this was significant at every visit except the second stable dose visit (p=0.106). Although providing no relevant efficacy information, results for the fampridine non-responders are also illustrated and show that there was, and could be, some significant improvement in leg strength when non-responder is treated with fampridine. This suggests that although a clinically meaningful response can be linked to about 37% of subjects treated with Fampridine-SR, additional subjects may have functional improvements on variables other than walking speed.

**Table 13. Summary of percent change in LEMMT at each double-blind visit by responder analysis grouping.**

| | | | | | |
|---|---|---|---|---|---|
| | | Summary Statistics Over Time | | | |
| | | Study day | | | |
| Treatment | | titration | 1st stbl | 2nd stbl | 3rd stbl |
| Placebo | Mean | -0.04 | -0.04 | -0.04 | -0.04 |
| | (SEM) | (0.035) | (0.042) | (0.039) | (0.042) |
| | N# | 46 | 46 | 46 | 45 |
| Fampridine | Mean | 0.12 | 0.10 | 0.09 | 0.10 |
| Non-responders | (SEM) | (0.028) | (0.033) | (0.036) | (0.038) |
| | N | 95 | 94 | 94 | 89 |
| Fampridine | Mean | 0.18 | 0.09 | 0.09 | 0.17 |
| Responders | (SEM) | (0.029) | (0.032) | (0.043) | (0.045) |
| | N | 58 | 58 | 58 | 58 |
| FR vs. Placebo | p-value^ | <0.001 | 0.023 | 0.106 | 0.004 |
| FR vs. FNR | p-value^ | 0.178 | 0.627 | 0.739 | 0.311 |
| FNR vs. PBO | p-value^ | <0.001 | 0.003 | 0.038 | 0.032 |
| **ABBREVIATIONS: FR=Fampridine Responders; FNR=Fampridine Non-responders.**<br>**\*\*: Significantly better than placebo and fampridine non-responders (p < 0.001 for each).**<br>**\*: Significantly better than fampridine non-responders.**<br>**#: The treatment sample sizes presented at individual time points may be smaller than those in the ITT population due to dropouts or missed assessments. Treatment sample sizes presented in the figure legend represent the number of ITT subjects. Sample sizes at individual time points may be smaller due to dropouts or missed assessments.**<br>**^: P-values from t-tests of the least-squares means using the mean square error via an ANOVA model with effects for responder analysis grouping and center.** | | | | | |

Figure 12 and Table 14, below, summarize the changes in Overall Ashworth Score at each double-blind visit by responder analysis grouping. The mean reduction from baseline (indicative of improvement) for the fampridine responders during the double-blind ranged from -0.18 to - 0.11 units compared to -0.11 to -0.06 for the placebo group. The fampridine responders were numerically superior to placebo but there was insufficient evidence to detect significant differences. Although appearing to provide little relevant efficacy information, results for the fampridine non-responders are also illustrated.

**Table 14. Summary of change in overall Ashworth score at each double-blind visit by responder analysis grouping.**

| | | | | | |
|---|---|---|---|---|---|
| | | Summary Statistics Over Time | | | |
| | | Study day | | | |
| Treatment | | titration | 1st stbl | 2nd stbl | 3rd stbl |
| Placebo | Mean | -0.06 | -0.11 | -0.06 | -0.13 |
| | (SEM) | (0.069) | (0.073) | (0.070) | (0.073) |
| | N# | 46 | 46 | 46 | 45 |
| Fampridine | Mean | -0.16 | -0.08 | -0.07 | 0.00 |

(continued)

| Summary Statistics Over Time | | | | | |
|---|---|---|---|---|---|
| | | Study day | | | |
| Treatment | | titration | 1st stbl | 2nd stbl | 3rd stbl |
| Non-responders | (SEM) | (0.044) | (0.053) | (0.054) | (0.056) |
| | N | 95 | 94 | 94 | 89 |
| Fampridine | Mean | -0.14 | -0.18 | -0.11 | -0.18 |
| Responders | (SEM) | (0.058) | (0.066) | (0.060) | (0.055) |
| | N | 58 | 58 | 58 | 58 |
| FR vs. Placebo | p-value^ | 0.343 | 0.374 | 0.717 | 0.680 |
| FR vs. FNR | p-value^ | 0.675 | 0.210 | 0.911 | 0.064 |
| FNR vs. PBO | p-value^ | 0.151 | 0.823 | 0.772 | 0.189 |

ABBREVIATIONS: FR=Fampridine Responders; FNR=Fampridine Non-responders.
**: Significantly better than placebo and fampridine non-responders (p < 0.001 for each).
*: Significantly better than fampridine non-responders.
#: The treatment sample sizes presented at individual time points may be smaller than those in the ITT population due to dropouts or missed assessments.
^: P-values from t-tests of the least-squares means using the mean square error via an ANOVA model with effects for responder analysis grouping and center.

Adverse events most commonly reported prior to treatment were accidental injury, reported by 12 (5.8%) subjects, nausea, reported by 9 (4.4%) subjects, and asthenia, diarrhea, and paresthesia, each reported by 8 (3.9%) subjects. Six (2.9%) subjects also reported headache, anxiety, dizziness, diarrhea, and peripheral edema. These adverse events are indicative of the medical conditions affecting people with MS.

Conclusions. The data does not appear to support either a number of anecdotal reports or expectations from preclinical pharmacology that doses higher than about 10 to 15 mg b.i.d., and even about 10 mg b.i.d., should be associated with greater efficacy. The data presented below in Table 15 support this, based on the new responder analysis methodology.

**Table 15. Comparison of 10 mg vs. 15 mg among Responders**

| | 10 mg (N=51) | 15 mg (N=50) |
|---|---|---|
| Responders N (%) | 18 (35.3) | 18 (36.0) |
| Average % CFB in Walk Speed: Mean (SD) | 27.6% (18.39) | 29.6% (22.43) |
| %Change in Walk Speed by Visit: minimum - maximum | 26% - 32% | 27% - 31% |
| Average SGI | 4.8 (1.09) | 4.7 (1.09) |
| Average Change in MSWS-12 * | -11.1 (21.9) | -7.8 (19.6) |
| * For the average change in the MSWS-12, a negative score is indicative of subjective improvement. | | |

A responder analysis based on consistency of improvement provides a sensitive, meaningful approach to measuring effects on the timed 25 foot walk and may be used as a primary endpoint for future trials. This data suggest that for responsive subjects (approximately 37%), treatment with fampridine at doses of 10-20 mg bid produces substantial and persistent improvement in walking.

*Efficacy.* There are no notable differences between 10 mg bid and 15 mg bid among subjects who respond to drug. In fact, the largest difference, favors the 10 mg bid group (see MSWS-12 result).

*Safety.* With respect to safety, there are three considerations: There was an apparent decline below baseline walking speed at the last visit on drug in the fampridine non-responders in the 10 mg bid and 20 mg bid groups, but not the 15 mg bid group. This may or may not be significant, but is not clearly dose related. There was an apparent rebound effect, with walking speed dropping below baseline, among fampridine treated subjects at the two week follow-up visit;

this occurred in the 15 and 20 mg but not the 10 mg bid group. Serious AE's were more frequent in the 15 mg and 20 mg bid groups 10% and 12% rates vs. 0% rate in 10 mg bid and 4% in placebo groups. This may or may not be significant, but the risk of potentially related SAEs, particularly seizures appears to be dose-related from all available data and based on mechanism of action. Based on this data, it would appear that a 10 mg bid dose is preferred because of its favorable risk to benefit ratio compared with the 15 and 20 mg doses.

Appendix B

Fig. 1

Fig. 2

**Adjusted geometric treatment means**

Fig. 3

**Treatment percentages**

Fig. 4

Fig. 5

Fig. 6

Fig. 7

treatment group percentages

Fig. 8

*Change from baseline in the MSWS-12 over the double-blind\**

**responder status means and standard error bars**

Non-responders (N=136)        Responders (N=62)

p = 0.020

*SGI over the double-blind*

**responder status means and standard error bars**

p = 0.004

Non-responders (N=141)        Responders (N=62)

*Change from baseline in the CGI over the double-blind*

**responder status means and standard error bars**

Non-responders (N=136)        Responders (N=62)

p = 0.056

\*\*Double-blind measurements at first and last stable dose visits only.
NNote: For the changes from baseline, a negative score is indicative of clinical benefit.
NNote: Some non-responders had no post-baseline data for a particular variable; so the sample sizes for the non-responders (with respect to that variable) may be less than the actual number of non-responders
NNote: The p-values comparing responders and non-responders are from ANOVA models with effects for responder status and center.
SSource: I:\Compounds\Fampridine-SR\MS\MS-F202\Stat\Tables\Efficacy\Post hoc\Validation of Responder Variable.sas

Fig. 9

Fig. 10

Fig. 11

Fig. 12

**APPENDIX C**

**Clinical Meaningfulness of Consistent Improvement on the Timed 25 Foot Walk (TW25) During Treatment with Fampridine**

Andrew Goodman[1], Ron Cohen[2], Lawrence Marinucci[2], Andrew Blight[2], and the Fampridine-SR Study Group

[1]Department of Neurology, University of Rochester Medical Center, Rochester, NY;
[2]Acorda Therapeutics Inc., Hawthorne, NY

**ABSTRACT**

Improvements in walking speed on the TW25 have been seen in controlled trials of fampridine (4-aminopyridine). The present study examined the relationship between changes in TW25, a Subject Global Impression (SGI), and the 12-Item MS Walking Scale (MSWS12). A total of 206 subjects with MS, with TW25 times of 8-60 seconds were enrolled in a 24-center, double-blind, placebo-controlled trial and randomized to one of four treatment groups: Fampridine (sustained release) 10, 15, 20mg BID or placebo, over a 14 week treatment period. In post-hoc analysis, a positive response on the TW-25 was defined as a walking speed during at least 3 of 4 visits during treatment that was faster than the maximum speed during the five off-drug visits. Response rate was higher in all fampridine groups (35, 36 and 39%) compared to placebo (9%, p<0.002). Responders showed significantly greater improvement in MSWS-12 scores and SGI scores during treatment compared to non-responders. The response criterion therefore identifies a meaningful change that affects subjects' impressions of their ambulatory disability. Adverse events and safety measures were consistent with previous experience.

## INTRODUCTION

Fampridine (4-aminopyridine, or 4-AP) is an experimental therapy for MS with a unique mechanism of action. At concentrations of 1-2 μM or less, fampridine appears to be a specific blocker of voltage dependent, neuronal potassium channels that affect conduction in demyelinated axons. It can restore action potential conduction in damaged, poorly myelinated nerve fibers, and it may also directly enhance synaptic transmission.[1,2] Fampridine-SR is a sustained release formulation of the drug. In previous clinical trials, treatment with fampridine has been associated with a variety of neurological benefits in people with MS including faster walking and increased strength [3-5].

Only a proportion of people with MS would be expected to be susceptible to these drug effects, given highly variable pathology. Currently, there is insufficient understanding to allow for pre-trial selection of individuals likely to respond. We have developed a novel statistical methodology which is based on the hypothesis that response to treatment should be reflected in a *consistent* improvement in walking speed. Here we address whether response on this criterion is a clinically meaningful outcome and whether treatment with fampridine significantly increases the likelihood of such a response.

## METHODS

## Figure 1   Study Design

A total of 206 subjects were enrolled in a 24-center, double-blind, placebo-controlled trial and randomized to one of four treatment groups: Fampridine (sustained release formulation) 10, 15, 20mg BID or placebo. A placebo run-in (2 wks) was followed by dose escalation (2 wks), stable treatment period (12 wks), down-titration (1 wk) and follow-up evaluation (2 wks).

### Inclusion Criteria

18-70 yr with definite MS (as defined by McDonald[6]), with adequate cognitive function and able to perform required study procedures, including a Timed 25-Foot Walk twice (required range to complete test at screening: 8 to 60 sec).

**Outcome Measures**

MS Functional Composite:

Timed 25-Foot Walk
9-Hole Peg Test
Paced Auditory Serial Addition Test (PASAT)-3"

Lower Extremity Manual Muscle Testing (LEMMT
Ashworth Score
Clinician Global Impression (CGI)
Subject Global Impression (SGI)
Multiple Sclerosis Quality of Life Inventory (MSQLI
12-Item Multiple Sclerosis Walking Scale (MSWS-12)[7].

**Response Criterion for Consistency of Improvement**

Consistency of improvement in walking speed was defined (post hoc) as achieving walking speed during at least three (i.e. the majority) of the four "on drug" visits that was faster than the maximum speed measured during the five "off-drug" visits (four prior, one follow-up). The selection of this criterion was supported by observation of the distribution of faster "on drug" visits between the fampridine and placebo-treated groups (Figure 2).

**Figure 2.** Histogram to show the proportion of subjects with a given number of "on drug" visits in which they achieved faster walking speed than the fastest of their five "off drug" visits. Fampridine-treated subjects showed more cases in which there were 3 or 4 faster on drug visits and less cases with no visits faster. The distribution of visits for the placebo-treated group is close to that predicted for sampling of random variability from visit to visit.

## Statistical Analysis

To determine if subjects with consistently improved walking speeds experienced meaningful improvements, statistical analyses were performed on two subjective variables (average SGI during the double-blind and the average change from baseline in MSWS-12 over the double-blind). Differences between response classifications (responders, vs. non-responders) were compared using an ANOVA model with effects for response classification and center.

The proportion of subjects with consistent improvements in walking speed (based on the retrospective definition) was analyzed by the Cochran-Mantel-Haenszel (CMH) test, controlling for center. For each dose group p-values (versus placebo) were Bonferroni-adjusted.

## RESULTS

### Table 1. Subject Disposition

|  | Placebo | 10 mg | 15 mg | 20 mg |
|---|---|---|---|---|
| **Randomized** | 47 | 52 | 50 | 57 |
| **Completed** | 45 | 50 | 49 | 51 |
| **Discontinued** | 2 | 2 | 1 | 6 |
| Adverse event | 1 | 0 | 1 | 5 |
| Non-compliant | 0 | 0 | 0 | 1 |
| Withdrew consent | 0 | 1 | 0 | 0 |
| Lost to follow-up | 1 | 1 | 0 | 0 |

## Baseline Demographics

There were no significant differences in baseline demographics (age, sex, race) or MS characteristics (diagnosis type, EDSS score, disease duration).

## Safety and Tolerability

- AEs occurred in 81% of placebo-treated subjects and 91% of fampridine-treated subjects. Most were mild to moderate in severity and transient.
- The most frequently reported AEs in the fampridine treatment groups were parathesia (10%), insomnia (8%), asthenia (7%), dizziness (7%), and nausea (6%).
- Serious adverse events, judged possibly or likely related to treatment, occurred in 3 subjects

  - Fampridine 20 mg bid: two subjects experienced a seizures during the trial, both cases following accidental overdoses (40 mg doses).
  - Fampridine 15 mg bid: one subject with altered mental state following overdose.

## Response Analysis

A subset of patients in each treatment group showed consistently improved walking speed while on drug: 8.5%, 35.3%, 36.0%, and 38.6% of the subjects in the placebo, 10 mg, 15 mg, and 20 mg b.i.d. treatment groups, respectively, as shown in Figure 3.

**Figure 3 Percentage of Subjects (ITT Population) with Consistent Improvement in Walking Speed (responders)**

Note: The p-values (versus placebo) are Bonferroni-adjusted. The unadjusted values were <0.002 for each group..

Given that there was little difference in responsiveness between the three doses examined, more detailed analyses compared the pooled fampridine-treated groups against the placebo-treated group. These analyses showed that subjects with consistently improved walking speeds averaged a net improvement of >20% in walking speed, during the treatment period and maintained a stable average level of improvement in walking speed at each visit, over the full course of treatment, as shown in Figure 4.

**FIGURE 4. Percent Change in Walking Speed at each Treatment Visit, by Response Analysis Grouping (Observed Cases, ITT population)**

**: Significantly better than placebo (p < 0.001 at each time point).
#: The treatment sample sizes presented in the figure legend represent the number of ITT subjects. Sample sizes at individual time points may be smaller due to dropouts or missed assessments.

**Subjective Correlates of Improved Walking**

Among the 205 ITT subjects, 62 experienced consistent improvements in walking speed, based on the post-hoc definition, while 143 did not. Analysis of the clinical meaningfulness of consistent improvements in walking speed indicate that consistency in walking speed improvements had clinical meaningfulness for the subjects in this study since these subjects had significantly better (higher) SGI scores and significantly better changes (reductions) in MSWS-12 perceived disability scores than those who did not (Figure 5).

**Figure 5   Analysis of the Clinical Meaningfulness of Consistent Improvements in Walking Speed (Observed Cases, ITT Population)**

*: Double-blind measurements at first and last stable dose visits only. For the changes from baseline, a negative score is indicative of clinical benefit.

Note: Some of the subjects with random improvements had no post-baseline data for a particular variable; so the sample sizes for is less than the actual number of subjects with random improvements in walking speed (N=143).

## CONCLUSIONS

These post-hoc analyses showed:

- the presence of a larger (x4) subset of subjects who responded to Fampridine-SR with a consistent improvement in walking speed in all fampridine-treated groups, compared to the placebo group.
- consistent improvement in walking speed was significantly associated with improved Subject Global Impression scores and MSWS-12 scores, indicating that consistent improvement in walking speed on the Timed Walk, associated with this treatment, is clinically meaningful to the subject.

These results support the continued investigation of Fampridine-SR for improvement of ambulatory function in MS through the prospective identification of a subset of subjects who respond on the Timed Walk test.

**REFERENCES**

1. Blight, A. R. (1989) Effect of 4-aminopyridine on axonal conduction block in chronic spinal cord injury. Brain Res Bull 22: 47-52.

2. Waxman SG. (1993) Aminopyridines and the treatment of spinal cord injury. J Neurotrauma 10: 19-24.

3. Davis, F.A., D. Stefoski, and J. Rush (1990) Orally administered 4-aminopyridine improves clinical signs in multiple sclerosis. Ann Neurol 27: 186-192.

4. Van Diemen, H.A.M. et al., (1992) The effect of 4-aminopyridine on clinical signs in multiple sclerosis: a randomized, placebo-controlled, double-blind, crossover study. Ann Neurol 32: 123-130.

5. Schwid, S.R., et al., (1997) Quantitative assessment of sustained-release 4-aminopyridine for symptomatic treatment of multiple sclerosis. Neurology 48: 817-821.

6. McDonald, W.I., et al. (2001) Recommended Diagnostic Criteria for Multiple Sclerosis: Guidelines from the International Panel on the Diagnosis of Multiple Sclerosis. Ann Neurol 50: 121-127.

7. Hobart J, Riazi A, Lampling D, Fitzpatrick R, Thompson A. (2003) Measuring the Impact of MS on Walking Ability. J Neurol 60:31-36.

**The MS-F202 Fampridine-SR Study Group:**

Barry Arnason, University of Chicago, Chicago, IL
Francois Bethoux, Cleveland Clinic Foundation, Cleveland, OH
Christopher Bever, University of Maryland at Baltimore, Baltimore, MD
James Bowen, University of Washington Medical Center, Seattle, WA
Anne Cross, Washington University-Barnes Jewish Hospital, St. Louis, MO
Corey Ford, University of New Mexico, Albuquerque, NM
Norman Kachuck, USC School of Medicine, Los Angeles, CA
Michael Kaufman, Carolinas Medical Center MS Clinic, Charlotte, NC
Lauren Krupp, SUNY Stony Brook, Stony Brook, NY
Thomas Leist, Thomas Jefferson University, Philadelphia, PA
John Lindsey, University of Texas-Houston, Houston, TX
Fred Lublin, Mt. Sinai School of Medicine - MS Center, New York, NY
Michelle Mass, Oregon Health Sciences University - MS Center, Portland, OR
Luanne Metz, Foothills Medical Centre, Calgary, AB
Aaron Miller, Maimonides Medical Center, Brooklyn, NY
Paul O'Connor, St. Michael's Hospital, Toronto, ON
Mary Ann Picone, Gimbel MS Center, Teaneck, NJ
Kottil Rammohan, Ohio State University, Columbus, OH
Marco Rizzo, Yale New Haven Hospital, New Haven, CT
Randall Schapiro, Fairview MS Center, Minneapolis, MN
Steven Schwid, University of Rochester, Rochester, NY
Craig Smith, Swedish Medical MS Center, Seattle, WA
Ben Thrower, MS Center at Shepherd Center, Atlanta, GA

((Footer bottom left))

Presented at the 130th Annual Meeting of the American Neurological Association San Diego, California, September 25-28, 2005.

((Footer bottom right))

Dr. Andrew D. Goodman is a consultant to Acorda Therapeutics, Inc. Financial support for this study was provided by Acorda Therapeutics, Inc.

**APPENDIX D**

**RESPONDER ANALYSIS**

**INTRODUCTION**

Acorda Therapeutics has sponsored two Phase 2, prospective, randomized, placebo-controlled clinical trials to evaluate the effects of Fampridine-SR on lower extremity motor function in people with multiple sclerosis (MS). Both trials demonstrated statistically significant improvement in leg strength, measured with the standard manual muscle test (LEMMT). Both trials showed improvements in walking speed based on the Timed 25 Foot Walk (TW25) which were statistically significant on the basis of post-hoc analyses. Increasing muscle weakness and ambulatory deficits are two of the most clinically significant aspects of this progressive disease. Fampridine is the first and only drug to date that has shown direct beneficial effects on functional deficits of walking and weakness in MS (as distinct from anti-inflammatory drugs used to promote recovery from acute relapse).

Acorda Therapeutics representatives met with the Division on July 1, 2004 to discuss the potential for proceeding to Phase 3 studies in light of the results of the most recent trial, MS-F202, a 211 patient, dose-comparison study. At this meeting, the Division presented the view that the data from MS-F202 demonstrated only a relatively small and transient effect of the drug on walking speed and that some additional measure of "clinical meaningfulness" of such changes on the TW25 would be required. It was suggested that a subject- or clinician-global measure could provide this role. It was also proposed that this role might be filled by the Multiple Sclerosis Walking Scale-12 (MSWS-12) a recently developed twelve item questionnaire that addresses walking function, and which was used in an exploratory way in study MS-F202. The Division also made clear the need to demonstrate the maintenance of effect over a 12-week treatment period.

Acorda Therapeutics has taken these recommendations into account and has revised its development plan to address the issues raised by the Division. Acorda now proposes to perform an adequately powered, double blind, randomized, placebo-controlled study to compare the effects of 10 mg b.i.d. Fampridine-SR against placebo on walking in patients with MS. The overall design of this study will be similar to that of study MS-F202, except for the dose comparison aspect of the previous trial, and will include a 14-week treatment period. As described in the attached protocol synopsis, and with regard to the expressed openness of the Division to considering such an approach, this study will employ a responder analysis as a primary endpoint. This form of analysis will facilitate evaluation of clinical meaningfulness of benefit and maintenance of effect in those subjects who respond to the drug. The following will first explain the rationale for the choice of response criterion and then illustrate in detail the application of the proposed analysis by means of a post-hoc analysis of study MS-F202.

The intent of this study and its analysis plan will be to show that there is a significant subset of subjects with ambulatory deficits due to MS who respond to Fampridine-SR with a meaningful increase in walking speed.

**RATIONALE**

Clinicians who regularly prescribe compounded fampridine for MS have reported that only a proportion of their patients appear to respond with clear clinical benefits, and that, in their judgment, this proportion may be around one third (personal communication with Acorda Therapeutics). This extent of responsiveness may be related to the proposed mechanism of action, which is the restoration of conduction in demyelinated axons via the blockade of voltage-dependent potassium channels. Only a proportion of MS patients would be expected to possess axons of appropriate functional relevance that are susceptible to these drug effects, given the highly variable pathology of the disease. Currently, there is insufficient understanding of the disease to allow for pre-trial selection of potentially responsive patients. However, the existence of a subset of patients who respond consistently to the drug can be supported by quantitative observations in studies MS-F201 and MS-F202.

Before treatment, the subjects in these two trials exhibited average walking speeds on the TW25 measure of approximately 2 feet per second (ft/sec). This is a significant deficit, since the expected walking speed for an unaffected individual is 5-6 ft/sec. Subjects in MS-F202 were selected for TW-25 walking time at screening of 8-60, which is equivalent to a range in speed of 0.42-3.1 ft/sec. Variability of functional status is an inherent characteristic of MS, and this can be seen in repeated measurement of walking speed over the course of weeks or months. At any of the three visits during the stable treatment period, 15-20% of placebo-treated subjects showed >20% improvement from baseline walking speed, a threshold chosen as one that indicates a true change in walking speed over background fluctuations. A larger proportion of the Fampridine-SR treated subjects showed such improvements, but this difference was not statistically significant, given the sample size and placebo response rate.

Given the often large variations in function experienced by people with MS, it is difficult for the subject or a trained observer to separate a treatment-related improvement from a disease-related improvement without the element of consistency over time. Consistency of benefit might therefore be expected to be a more selective measure of true treatment effect than magnitude of change. Based on this rationale, the responses of the individual subjects in the MS-F202 trial were examined for the degree to which their walking speed showed improvement during the double-blind treatment period and returned towards pre-treatment values after they were taken off drug, at follow-up. This subject-by-subject examination yielded a subgroup of subjects whose pattern of walking speed over time appeared to be consistent with a drug response. This led to the analysis illustrated in **Figure 1.** This compares the placebo and Fampridine-SR treated groups with respect to the number of visits during the double-blind treatment period in which walking speed on the TW25 was faster than the maximum speed out of all five of the non-treatment visits (four visits prior to randomization and one follow-up visit after the drug treatment period).

The placebo-treated group showed a clear pattern of exponential decline in numbers of subjects with higher numbers of "positive" visits. This is what would be expected from a random process of variability (a simple computer simulation of the random sampling process involved shows the same pattern of response - **Fig. A1** in the Supplemental Tables and Figures). In contrast, the pattern of response in the Fampridine-SR treated group strongly diverged from this distribution; much larger numbers of Fampridine-SR treated subjects showed three or four visits with higher walking speeds than the maximum speed of all five non-treatment visits and less than half of the expected proportion had no visits with higher speeds. These results indicate that there was a sub-population of subjects in the Fampridine-SR treated group that experienced a consistent increase in walking speed related to treatment.

**Fig. 1** Histogram to show the number of treatment visits at which subjects showed faster walking speed on the timed 25 foot walk than at all of the five non-treatment visits.

This analysis suggests that a relatively highly selective criterion for a likely treatment responder would be: a subject with a faster walking speed for at least three (i.e., three or four) of the four visits during the double blind treatment period compared to the maximum value for all five of the non-treatment visits. The four visits before initiation of double-blind treatment provide an initial baseline against which to measure the consistency of response during the four treatment visits. The inclusion of the follow-up visit as an additional component of the comparison was found valuable primarily in excluding those subjects who did not show the expected loss of improvement after coming off the drug. These are likely to be subjects who happened by chance to have improved in their MS symptoms around the time of treatment initiation, but whose improvement did not reverse on drug discontinuation because it was actually unrelated to drug. Thus, incorporating the follow-up visit as part of the criterion may help to exclude false positives, if the TW25 speed remains high at follow-up.

In study MS-F202, this responder criterion was met by 8.5%, 35.3%, 36.0%, and 38.6% of the subjects in the placebo, 10 mg, 15 mg, and 20 mg b.i.d. treatment groups, respectively, showing a highly significant and consistent difference between placebo and drug treatment groups. Given that there was little difference in responsiveness between the three doses examined, more detailed analyses were performed comparing the pooled Fampridine-SR treated groups against the placebo-treated group. The full results of this analysis for study MS-F202 are described in the following sections. These show that the responder group so identified experienced a >25% average increase in walking speed over the treatment period and that this increase did not diminish across the treatment period. The responder group also showed an increase in Subject Global Impression score and an improvement in score on the MSWS-12. If this same pattern of improvement on the secondary endpoints is confirmed in the prospectively designed studies, the clinical meaningfulness of the response definition will be supported.

It is important to note that this responder criterion selects subjects who show a pattern of change that is consistent with a treatment response, but does not define the full characteristics of that response. The criterion itself does not specify the amount of improvement nor does it specify that the improvement must be stable over time. For example, a progressive decline in effect during the course of the study period, even one resulting in speeds slower than the maximum non-treatment value, would not be excluded by the criterion; as a specific example, changes from the maximum non-treatment value of, respectively, +20%, +5%, + 1% and -30% during the double blind treatment period would qualify as a response under the criterion, but would actually show a net negative average change for the entire period, poor stability and a negative endpoint. Post-hoc analyses of studies MS-F202 and MS-F201 indicate that we may expect responders defined by consistency of effect also to demonstrate increased magnitude and stability of benefit, but this must be determined in a well-controlled trial in which this responder criterion is pre-defined.

**RESPONDER ANALYSIS APPLIED TO STUDY MS-F202**

Given that there was little difference in responsiveness between the three drug doses examined in Study MS-F202 (see **Table A1** in the Supplemental Tables and Figures) the following analysis primarily compares the pooled Fampridine-SR treated groups against the placebo-treated group.

The number of subjects who met the responder criterion in the pooled Fampridine-SR treated group was 58 (36.7%) compared to 4 (8.5%) in the placebo-treated group, and this difference was statistically significant (p<0.001) as shown in **Table 1.** Fig. A2 (see Supplemental Tables and Figures) illustrates the mean changes walking speeds by visit for these two groups.

| Table 1 (MS-F202) Number and Percentage of Responders (ITT Population) | | | |
|---|---|---|---|
| **Parameter** | **Statistic** | **Placebo (N=47)** | **Fampridine-SR Pooled (N=158)**\*\* |
| Responder Status | | | |
| Non-Responder | N (%) | 43 (91.5) | 100 (63.3) |
| Responder | N (%) | 4 (8.5) | 58 (36.7) |
| | Treatment p-value\* | <0.001 | |
| \*From Cochran-Mantel-Haenszel (CMH) test, controlling for center; using general association statistic. <br> \*\*Two randomized subjects who did not return for on-treatment efficacy assessments were considered non-responders <br> Source I:\Compounds\Fampridine-SR\MS\MS-F203\Stat\Exploratory\Protocol Devepment\Table 05_Secondary Responder Status sas | | | |

This indicates that the chances of a more favorable outcome, based on consistency of improvement in walking speed, is much higher in the Fampridine-SR treated than in the placebo-treated group.

**Figure 2** compares changes in walking speed for the Responder and Non-Responder groups between the Fampridine-SR and placebo-treated groups. The Fampridine-SR Responder group showed an average increase in speed of 0.49 ft/sec compared to 0.01 ft/sec in the two Non-Responder groups. The four Placebo Responder subjects showed an intermediate average change in walking speed (0.33 ft/sec) but there were too few subjects in this group for meaningful comparison. There was no difference between the groups at baseline in baseline walking speed, demographics, or neurological characteristics (see Table A2, A3, and A4 in the Supplemental Tables and Figures).

**Fig 2.** Average change in walking speed during the double blind treatment period, compared to average pre-randomization value, by responder classification.

The average percent improvement from baseline in walking speed during the double blind period was 27% for the Fampridine-SR Responder groups, compared to 1 and 2% in the Placebo and Fampridine-SR Non-Responders, respectively, as shown in **Figure 3**.

**Fig. 3** Average percent change in walking speed during the double-blind treatment period, compared to average pre-randomization value, by responder classification.

Not only was the magnitude of effect on average walking speed substantial in the Fampridine-SR Responder group, but the effect was stable and the confidence intervals well separated from the non-responder groups over the full 14-week treatment period, as shown in **Figure 4.**

Note 1: The treatment sample sizes presented in the figure legend represent the number of ITT subjects. Sample sizes at individual time points may be smaller due to dropouts or missed assessments.

**Fig. 4** Percent change in walking speed, compared to average pre-randomization values, by responder classification at each study visit during the double-blind treatment period. For clarity, the upper graph shows only the Fampridine-SR Responder group and the Fampridine-SR and Placebo Non-Responders. The lower graph includes the Placebo Responders, showing the large confidence intervals which would otherwise obscure the display of confidence intervals for the other groups.

The response criterion also allows us to explore the beneficial effects of this change as reported by the subjects on two global measures, the Subject Global Impression (SGI) and Multiple Sclerosis Waling Scale-12 (MSWS-12), as illustrated in **Figures 5 and 6,** below. The Responders showed a higher average score on the SGI during the double-blind treatment period. These SGI data indicate that Responders were able to distinguish a positive benefit associated with their improvement in walking speed. A larger sample would be required to confirm this difference statistically given the highly variable nature of responses on these variables (see Fig. A3 and A4 in the Supplemental Tables and Figures).

**Fig. 5** Average Subject Global Impression score by responder classification during the double-blind treatment period. A score of 4 is a response of "neutral/mixed" on a 7-point Terrible(1) to Delighted (7) scale.

Clinical meaningfulness of the responder criterion is also supported by the fact that Responders showed greater improvement on the MSWS-12 questionnaire, as shown in **Figure 6,** below. Note that the MSWS-12 was only applied at Visit 0 (before placebo run-in) and at the first and last stable dose visits in study MS-F202.

Fig. 6 Average change from baseline (Visit 0) in MSWS-12 score during the double-blind treatment period, by responder classification. The MSWS-12 score has a maximum value of 100 for the most severe impact of MS upon ambulatory function in daily life functions.

In summary, this responder analysis illuminates characteristics of the response that are not clear from analysis of the whole treatment population. The benefit on walking in Responders appears to be both substantial, with an average improvement of more than 25%, and quantitatively consistent over the 3-month period of the treatment. In addition, the Responders appear to register their impressions of clinical benefit on both the SGI and MSWS-12.

## PROPOSAL FOR NEXT PHASE 2 STUDY

With the benefit of these analyses applied retrospectively to study MS-F202 and also to study MS-F201 (see **Table A5** in the Supplemental Tables and Figures) Acorda Therapeutics proposes to perform a new prospective study, based closely on the design of MS-F202. A dose of 10 mg b.i.d. has been chosen since there was little evidence of a dose-related increase in response. Therefore, the lowest dose has been chosen in order to minimize adverse events, including, hopefully, the risk of seizures.

As described in the attached protocol synopsis, this study will declare a primary endpoint of response on the pre-defined responder analysis. In addition, as part of the secondary analyses, the clinical meaningfulness of these improvements will be assessed by the magnitude of improvement in speed on the TW-25 and with the SGI and MSWS-12 global instruments. Further clinical benefit will be assessed by use of the LEMMT to measure lower extremity muscle strength.

This new study is expected to demonstrate significance on the responder analysis, based on consistency of response over time. It is also expected to demonstrate sustained increase in walking speed in Responders compared to Non-Responders. The study may also demonstrate improvement in Responders on the global measures (SGI and MSWS-12). In the event that the study is not able to demonstrate clear separation of Responders on these global measures, it is expected to provide additional information on variability and sensitivity of the instruments to changes in walking speed. This will be useful in further assessing the potential validity of these measures in this context and may permit appropriate power calculations for subsequent studies.

**SUPPLEMENTAL TABLES AND FIGURES**

**Responder Rate for Individual Dose Groups (MS-F202)**

The response rate of Fampridine-SR treated subjects seen in the pooled analysis was consistent within the three drug groups, as shown in **Table A1.** Likewise, individual dose group analyses of magnitude of effect, maintenance of effect and linked global effects are broadly consistent, though the small sample sizes limit the significance of the observed effects.

| Table A1 (MS-F202) Number and Percentage of Responders by Randomized Group (ITT Population) | | | | | |
|---|---|---|---|---|---|
| **Parameter** | **Statistic** | **Placebo (N=47)** | **10mg b.i.d. (N=51)** | **15mg b.i.d. (N=50)** | **20mg b.i.d. (N=57)** |
| Responder | N (%) | 4 (8.5) | 18 (35.3) | 18 (36.0) | 22 (38.6) |
| Non-Responder | N (%) | 43 (91.5) | 33 (64.7) | 32 (64.0) | 35 (61.4) |

**Baseline Characteristics of Responder Groups (MS-F202)**

There were no significant differences between Responder, Non-responder and Placebo groups in terms of baseline characteristics or demographics, as shown in the following tables (A2-A4).

| Table A2 (MS-F202) Baseline Walking Speed (ft/sec) by Responder Status (ITT Population) | | | | | | |
|---|---|---|---|---|---|---|
| **Parameter** | **Statistic** | **Placebo Non-Responders (N=43)** | **Fampridine Non-Responders (N=100)** | **Placebo Responders (N=4)** | **Fampridine Responders (N=58)** | **Total (N=205)** |
| Baseline Walk Speed | MEAN (STD) | 1.84 (0.88) | 1.92 (0.93) | 2.15 (0.87) | 2.04 (0.77) | 1.94 (0.87) |
| | MEDIAN | 1.63 | 1.90 | 2.19 | 2.15 | 1.91 |
| | RANGE | (0.4, 4.8) | (0.3, 6.2) | (1.1, 3.2) | (0.6, 3.8) | (0.3, 6.2) |
| | Treatment p-value* | 0.68 | | | | |
| * From ANOVA with effects for responder group and center. I:\Compounds\Fampridine-SR\MS\MS-F203\Stat\Exploratory\Protocol Development\Library03\Revised Baseline_ Baseline WS by Responder Status sas | | | | | | |

| Table A3 (MS-F202) Baseline Demographics by Responder Status (Safety Population) | | | | | | |
|---|---|---|---|---|---|---|
| **Parameter** | **Statistic** | **Placebo Non-Responders (N=43)** | **Fampridine Non-Responders (N=101)** | **Placebo Responders (N=4)** | **Fampridine Responders (N=58)** | **Total (N=206)** |
| **GENDER:** | | | | | | |
| **Male** | N (%) | 20 (46.5) | 38 (37.6) | 0 (0.0) | 17 (29.3) | 75 (36.4) |
| **Female** | N (%) | 23 (53.5) | 63 (62.4) | 4 (100.0) | 41 (70.7) | 131 (63.6) |
| | Treatment p-value* | 0.138 | | | | |

(continued)

| Table A3 (MS-F202) Baseline Demographics by Responder Status (Safety Population) | | | | | | |
|---|---|---|---|---|---|---|
| **Parameter** | **Statistic** | **Placebo Non-Responders (N=43)** | **Fampridine Non-Responders (N=101)** | **Placebo Responders (N=4)** | **Fampridine Responders (N=58)** | **Total (N=206)** |
| **GENDER:** | | | | | | |
| | | | | | | |
| **AGE** | N | 43 | 101 | 4 | 58 | 206 |
| | Mean (SD) | 48.4 (8.8) | 50.0 (9.2) | 56.0 (9.3) | 50.0 (7.9) | 49.8 (8.7) |
| | Median | 49.0 | 50.0 | 58.5 | 50.5 | 50.0 |
| | (Min, Max) | (28, 69) | (28, 69) | (43, 64) | (30, 68) | (28, 69) |
| | Treatment p-value* | 0.358 | | | | |
| | | | | | | |
| **RACE:** | | | | | | |
| **Caucasian** | N (%) | 41 (95.3) | 94 (93.1) | 3 (75.0) | 52 (89.7) | 190 (92.2) |
| **Black** | N (%) | 1 (2.3) | 6 (5.9) | 1 (25.0) | 2 (3.4) | 10 (4.9) |
| **Asian/ Pacific Islander** | N (%) | 0 (0.0) | 0 (0.0) | 0 (0.0) | 1 (1.7) | 1 (0.5) |
| **Hispanic** | N (%) | 0 (0.0) | 1 (1.0) | 0 (0.0) | 2 (3.4) | 3 (1.5) |
| **Other** | N (%) | 1 (2.3) | 0 (0.0) | 0 (0.0) | 1 (1.7) | 2 (1.0) |
| | Treatment p-value* | 0.466 | | | | |
| | | | | | | |
| **WEIGHT** | N | 43 | 100 | 4 | 57 | 204 |
| | Mean (SD) | 75.2 (16.1) | 73.8 (17.8) | 60.8 (9.6) | 76.0 (16.1) | 74.4 (16.9) |
| | Median | 75.5 | 73.6 | 63.7 | 72.9 | 73.3 |
| | (Min, Max) | (50, 118) | (41, 145) | (47, 68) | (50, 118) | (41, 145) |
| | Treatment p-value* | 0.344 | | | | |
| *Note: p-values for gender and race from a chi-square test; p-values for age and weight from ANOVA. I:\Compounds\Fampridine-SR\MS\MS-F203\Stat\Exploratory\Protocol Development\Library03\Demographics by Responder Status.sas | | | | | | |

Table A4 (MS-F202)
Key Baseline Neurological Characteristics by Responder Status (Safety Population)

| Parameter | Statistic | Placebo Non-Responders (N=43) | Fampridine Non-Responders (N=101) | Placebo Responders (N=4) | Fampridine Responders (N=58) | Total (N=206) |
|---|---|---|---|---|---|---|
| DIAGNOSIS: | | | | | | |
| Relapsing Remitting | N (%) | 13 (30.2) | 22 (21.8) | 0 (0.0) | 12 (20.7) | 47 (22.8) |
| Primary Progressive | N (%) | 10 (23.3) | 24 (23.8) | 2 (50.0) | 15 (25.9) | 51 (24.8) |
| Secondary Progressive | N (%) | 20 (46.5) | 55 (54.5) | 2 (50.0) | 31 (53.4) | 108 (52.4) |
| | Treatment p-value* | 0.726 | | | | |
| | | | | | | |
| EDSS score | N | 43 | 101 | 4 | 58 | 206 |
| | Mean (SD) | 5.8 (1.0) | 5.8 (0.9) | 6.1 (0.3) | 5.7 (1.0) | 5.8 (1.0) |
| | Median | 6.0 | 6.0 | 6.0 | 6.0 | 6.0 |
| | (Min, Max) | (3,7) | (3, 7) | (6, 7) | (3, 7) | (3, 7) |
| | Treatment p-value* | 0.665 | | | | |

*Note: p-values for diagnosis type from a chi-square test; p-values for EDSS from ANOVA.
I:\Compounds\Fampridine-SR\MS\MS-F203\Stat\Exploratory\Protocol Development\Library03\Demographics by Responder Status.sas

**Equivalent Responder Analysis in Study MS-F201**

In this analysis, data from Study MS-F201 was treated in an approximately equivalent manner to the approach for MS-F202, using the first four visits on drug (one visit per week, with weekly dose escalation through 10, 15, 20 and 25 mg b.i.d.) and comparing to 5 visits "off drug" before randomization. This was a smaller study, with a very small placebo group, but the results of the responder analysis were largely consistent with Study MS-F202, particularly with regard to the percent frequency of response in the Fampridine-SR and placebo-treated groups. The MSWS-12 measure was not used in this study.

Table A5 (MS-F201)
Summary of results by clinical status (observed cases, ITT population)

| | | Responder Status | |
|---|---|---|---|
| Fampridine-SR | Statistic | Responder | Non-Responder |
| Avg. Change in Walking Speed (ft/sec) | **N (%)** N Mean (SD) | **10 (40.0)** 10 0.37 (0.17) | **15 (60.0)** 15 0.11 (0.28) |
| Change in Walking Speed at Endpoint (ft/sec) | N Mean (SD) | 10 0.40 (0.28) | 15 0.11 (0.37) |
| Average SGI | N Mean (SD) | 10 4.4 (1.03) | 15 4.1 (0.84) |

(continued)

| Placebo | Statistic | Responder | Non-Responder |
|---|---|---|---|
| | **N (%)** | **1 (9.1)** | **10 (90.9)** |
| Avg. Change in Walking Speed (ft/sec) | N | 1 | 10 |
| | Mean (SD) | 0.66 (NA) | -0.07 (0.26) |
| Change in Walking Speed at Endpoint (ft/sec) | N | 1 | 10 |
| | Mean (SD) | 0.99 (NA) | -0.06 (0.34) |
| Average SGI | N | 1 | 10 |
| | Mean (SD) | 5.0 (NA) | 4.6 (0.74) |

**Probability of Treatment Visits with Faster Walking Speed (MS-F202)**

**Fig. A1.** Histogram to compare the observed frequency of faster walking speed during treatment visits in the placebo group (as shown in Figure 1) compared with a computer model simulation of the underlying sampling procedure. The simulation involved computer generation of 100,000 trains of 9 random numbers and testing the frequency with which numbers 5-8 were larger than the maximum of the numbers 1-4 and 9 in each train. The model predicts an exponential decline in probability of larger numbers of positive visits, and the observed values were close to this distribution. The slightly higher observed values may relate to the trend for increase in mean walking speed over the course of the trial in the placebo group (see **Fig. A2**).

**Change in Walking Speed Over Time of Treatment - Pooled Fampridine-SR Group (MS-F202)**

**Treatment means over time**

Placebo (N=47)
Fampridine (N=158)

| Summary Statistics Over Time | | | | | | |
|---|---|---|---|---|---|
| | | Study Day | | | |
| Treatment | | titration | 1st stable | 2nd stable | 3rd stable |
| Placebo | Mean | 0.02 | 0.02 | 0.02 | 0.04 |
| | (SD) | (0.283) | (0.399) | (0.359) | (0.349) |
| | N* | 47 | 46 | 46 | 45 |
| Fampridine | Mean | 0.24 | 0.17 | 0.17 | 0.08 |
| | (SD) | (0.365) | (0.379) | (0.417) | (0.514) |
| | N | 155 | 152 | 150 | 147 |

**Fig. A2.** Plot and table to compare the average change in walking speed at each treatment visit for the pooled Fampridine-SR group with the change in the Placebo group. Changes in this original analysis were measured from the average during the placebo run-in visits. This analysis gives the impression of an initial improvement in speed which declines over time, losing significance by the last study visit. The responder analysis (as in **Fig. 4**, above) shows that the apparent decline in improvement was actually due to an early transient improvement and a subsequent drop on the last visit in the Fampridine-SR Non-Responder group. The Fampridine-SR Responder subjects, with a consistent improvement throughout treatment were responsible for the great majority of the overall treatment effect. Hence the temporal variability seen here in the analysis of means appears to be quite separate from the marked treatment-related improvement in speed. Note that any subjects who actually showed a pattern of response similar to the Fampridine-SR group mean would almost certainly meet the responder criterion and would be included in the Responder category.

**Variability of Global Measures (MS-F202)**

The data from study MS-F202 provide only limited information on the two subject global measure (SGI and MSWS-12) but sufficient to illustrate the highly variable nature of responses on these instrument. Even in the placebo-treated group, both SGI and MSWS-12 showed a much higher range of spontaneous variability between study visits than the TW25 or lower extremity strength, which are more direct and objective measures of function (see **Figure A3** below).

**Fig. A3.** Scatterplots showing the variability of four different measures of lower extremity function between baseline measurements and measurements after 6 weeks on double-blind study (Study Day 56) for the Placebo-treated group in Study MS-F202. Both the SGI and the MSWS-12 show more variability (spread) and less overall stability (slope) with respect to baseline than the walking speed on the TW25 and leg strength on the LEMMT scale. Note that the SGI is restricted to integer values, and the plot indicates the range of values and the frequency by number at points with frequency >1.

Further analysis of the MSWS-12 scores in placebo-treated subjects, is provided in **Figure A4.** This shows that the individual's assessment of the impact of ambulatory deficits on everyday function is remarkably variable for a given level of objective deficit, as defined by the timed walk. This is likely to be reflective of wide disparities in subject expectation and adopted lifestyles, which can allow one subject to feel severely disabled by a relatively small decline in motor function while another may adapt lifestyle and expectation to a persistent, severe ambulatory deficit, and might therefore take some considerable time to re-adapt to or even recognize a new level of function.

**Fig. A4.** Scatterplot to show the relationship between the MSWS-12 score and walking speed, measured with the TW25 for the entire placebo-treated group over all four study visits at which the instrument was used. The MSWS-12 score shows increasing severity of impact on walking speed with higher numbers. A normal walking speed is 5.5-6 ft/sec.

**Appendix E**

# Responder Analysis of Walking Speed Applied to a Trial of Fampridine in Subjects with MS

POSTER NUMBER 169

Ron Cohen[1], Lawrence N. Marinucci[1], Andrew R. Blight[1], Mitchell A. Katz[1], Andrew D. Goodman[2], Jeffrey A. Cohen[3]

[1]Acorda Therapeutics Inc., Hawthorne, NY; [2]Department of Neurology, University of Rochester Medical Center, Rochester, NY; [3]Department of Neurology, The Cleveland Clinic Foundation, Cleveland OH

## INTRODUCTION

Fampridine (4-aminopyridine, "4-AP") is a potential therapy for MS with a unique mechanism of action. At concentrations of 1-2μM or less, fampridine appears to be a specific blocker of voltage dependent, neuronal potassium channels that affect conduction in demyelinated axons. Fampridine has been shown to restore action potential conduction in damaged, poorly myelinated nerve fibers, and it may also directly enhance synaptic transmission. In previous clinical trials, treatment with fampridine has been associated with a variety of neurological benefits in people with MS including faster walking and increased strength, as measured by standard neurological assessments.

Clinicians who regularly prescribe compounded fampridine (4-aminopyridine) for MS have reported that a subset of their patients appear to respond with clear clinical benefits. The extent of responsiveness may be related to the proposed mechanism of action, which is the restoration of conduction in demyelinated axons via blockade of potassium channels. Only a proportion of MS patients would be expected to have axons of appropriate functional relevance that are susceptible to these drug effects, given the highly variable pathology of the disease. Currently, there is insufficient understanding of that pathology to allow for pre-trial selection of responsive patients. The focus of this poster is to present the results of a post-hoc analysis of fampridine responders.

## METHODS

**FIGURE 1. Study Design**

### Prospective Primary Endpoint

Percent improvement in average walking speed during the stable dosing period (visits 7-9), relative to the baseline, placebo run-in period (visits 1-2), using the Timed 25 Foot Walk from the Multiple Sclerosis Functional Composite (MSFC).

### Response Criterion

A positive response to treatment was defined as achieving walking speed during at least three of the four "on drug" visits that was faster than the maximum speed measured during the five "off-drug" visits (four prior, one follow-up). The selection of this criterion was supported by observation of the distribution of faster "on drug" visits between the fampridine and placebo-treated groups (Figure 2).

## RESULTS

**TABLE 1. Subject Disposition**

| | | | | |
|---|---|---|---|---|
| Randomized | 47 | 52 | 50 | 57 |
| Completed | 45 | 50 | 49 | 51 |
| Discontinued | 2 | 2 | 1 | 6 |
| Adverse event | 1 | 0 | 1 | 5 |
| Non-compliant | 0 | 0 | 0 | 1 |
| Withdrew consent | 0 | 1 | 0 | 0 |
| Lost to follow-up | 1 | 1 | 0 | 0 |

**FIGURE 2. Distribution of "faster" visits**

Number of Treatment Visits with Faster Walking Speed

### Secondary Endpoints:
- Lower Extremity Manual Muscle Test (LEMMT) for knee flexors and extensors, hip adductors and ankle flexors, left and right sides
- 9-Hole Peg Test
- Paced Auditory Serial Addition Test 3"
- Ashworth Score for Spasticity
- 12-item Multiple Sclerosis Walking Scale (MSWS-12)
- Clinician Global Impression of Change
- Subject Global Impression
- Multiple Sclerosis Quality of Life Inventory

### Inclusion Criteria

18-70 yr with definite MS (as defined by McDonald), with adequate cognitive function and able to perform required study procedures; including a Timed 25-Foot Walk twice (required range to complete test at screening: 8 to 60 sec).

### Statistical Analysis

The pre-planned primary efficacy variable, percent change in average walking speed during the 12-week stable dose period, was analyzed by ANOVA with effects for treatment and center; Dennett adjusted p-values are presented for the comparisons vs. placebo.

The proportion of responders (based on the retrospective definition) was analyzed by the Cochran-Mantel-Haenszel (CMH) test, controlling for center; Bonferroni adjusted p-values are presented for the comparisons vs. placebo.

## Baseline Demographics

There were no significant differences in baseline demographics (age, sex, race) or MS characteristics (diagnosis type, EDSS score, disease duration).

### Safety and Tolerability
- AEs occurred in 81% of placebo-treated subjects and 91% of fampridine-treated subjects
- Most AEs were mild to moderate in severity and were transient.
- The most frequently reported AEs in the fampridine treatment groups were paresthesia (10%), insomnia (8%), asthenia (7%), dizziness (7%), and nausea (6%).
- Serious adverse events, judged possibly or likely related to treatment, occurred in 3 subjects
  - Fampridine 20 mg bid: two subjects experienced a seizure during the trial, one of these experienced two seizures; in both cases following accidental overdoses (40 mg doses).
  - Fampridine 15 mg bid: one subject with altered mental state following overdose

## Efficacy

### Prospective Primary Endpoint Analysis

Although there were positive trends across the treatment groups, improvement in the primary efficacy variable, percent change in average walking speed during the stable dose treatment period, was not statistically significant by the planned ANOVA analysis (Figure 3).

**FIGURE 3. Percent change in average walking speed during the 12-week stable dose period (observed cases, ITT population)**

Adjusted Geometric Treatment Means

### Responder Analysis

Post-hoc analyses of the data revealed a subset of patients who showed consistently improved walking speed while on drug: 8.5%, 35.3%, 36.0%, and 36.8% of the subjects in the placebo, 10mg, 15 mg, and 20 mg b.i.d. treatment groups, respectively (Figure 4).

**FIGURE 4. Percentage of responders by treatment group (ITT population)**

Given that there was little difference in responsiveness between the three doses examined, more detailed analyses compared the pooled fampridine-treated groups against the placebo-treated group and showed that subjects qualifying as responders:
- were more than four times more likely to have been treated with fampridine than placebo (35.7% vs. 8.5%, respectively; p < 0.001)
- showed a net improvement of >25% in walking speed, during the treatment period
- maintained a stable average level of improvement in walking speed at each visit, over the full course of treatment (Figure 5)

**FIGURE 5. Percent change in average walking speed at each treatment visit, by response analysis grouping (observed cases, ITT population)**

Mean Percent Change — Time on Double-Blind Treatment

## SECONDARY ENDPOINTS

Of the secondary endpoints included, LEMMT showed statistically significant improvement in the fampridine responder group compared to placebo. The MSWS-12 and SGI showed statistically significant improvement in responders compared to non-responders, indicating the selected walking speed response represents a clinically meaningful impact on function. This aspect of the response analysis will be considered in detail elsewhere.

## CONCLUSION

- Using the planned analysis, there was an increase in mean walking speed during the stable dose period for all fampridine dose groups compared to placebo, but this was not statistically significant.
- A post-hoc analysis showed the presence of a subset of subjects who responded to treatment with a consistent improvement in walking speed.
- Improvement in walking speed among responders was stable across the 14 weeks of treatment, large (>25% on average) and seen at significantly higher frequency (>4x) in all fampridine treated groups compared to the placebo group.

## REFERENCES

1. Blight, A. R. (1989) Effect of 4-aminopyridine on axonal conduction block in chronic spinal cord injury. Brain Res Bull 22: 47-52.
2. Waxman, S.G. (1993) Aminopyridines and the treatment of spinal cord injury. J Neurotrauma 10
3. Davis, F.A., G. Stefoski, and J. Rush (1990) Orally administered 4-aminopyridine improves clinical signs in multiple sclerosis. Ann Neurol 27: 186-192.
4. Van Diemen, H.A.M. et al. (1992) The effect of 4-aminopyridine on clinical signs in multiple sclerosis; a randomized, placebo-controlled, double-blind, crossover study. Ann Neurol 32: 123-130.
5. Schwid, S.R., et al. (1997) Quantitative assessment of sustained-release 4-aminopyridine for symptomatic treatment of multiple sclerosis. Neurology 48: 817-821.
6. Personal communication to Acorda Therapeutics.
7. McDonald, W.I., et al. (2001) Recommended Diagnostic Criteria for Multiple Sclerosis: Guidelines from the International Panel on the Diagnosis of Multiple Sclerosis. Ann Neurol 50: 121-127.

**EP 1 940 285 B1**

**Claims**

1. A computer implemented method for analyzing the therapeutic effect of a treatment of patients in a clinical environment comprising the following steps:

   (i) identifying a plurality of records relating to patients in a clinical database, said records comprising measurements for the patients relating to tests administered during an off-treatment period and an on-treatment period;
   (ii) identifying at least one test in said plurality of records relating to k measurements of each patient during an off-treatment period;
   (iii) identifying at least one test in said plurality of records relating to j measurements of each patient during an on-treatment period;
   (iv) identifying a best measurement from said k measurements of each patient during said off-treatment period;
   (v) performing a statistical distribution assessment on said plurality of records to identify likelihoods of a plurality of subsets of said j on-treatment measurements exceeding said best measurement, wherein the statistical distribution assessment uses a computer model based on random number generation or is derived from executing a range disparity distribution;
   (vi) selecting a threshold likelihood, corresponding to the likelihood of a number of j measurements exceeding said best measurement, said number of measurements exceeding said best measurement indicating an improved performance and thereby a treatment response in a patient;
   (vii) identifying one or more acceptable subsets from the plurality of subsets of j on-treatment measurements with respective likelihoods that are not above the threshold likelihood;
   (viii) comparing said j on-treatment measurements with said best measurement; and
   (ix) selecting the patients who have the number of j measurements exceeding said best measurement as one of the acceptable subsets.

2. The computer implemented method of claim 1, further comprising administering the test to selected patients after the treatment period, wherein the patients are selected based on the further exhibition of an improved performance during a majority of the tests administered during the treatment period as compared to the test administered after the treatment period.

3. The computer implemented method of claim 1 or claim 2, wherein the patients are afflicted by a neurological disorder.

4. The computer implemented method of claim 3, wherein the neurological disorder is multiple sclerosis, spinal cord injuries, Alzheimer's disease or ALS.

5. The computer implemented method of claim 4, wherein the neurological disorder is multiple sclerosis and the computer implemented method further comprises the step of establishing a baseline comparability among groups of said patients who exhibited improved response by analyzing baseline demographic variables, key neurological characteristics, and efficacy variables at said baseline.

6. A computer program for use with a computer system, for analyzing the therapeutic effect of a treatment of patients in a clinical environment, the computer program comprising:
   a computer readable medium containing thereon instructions operative to control the operation of a computer to perform the steps of:

   (i) identifying a plurality of records relating to patients in a clinical database, said records comprising measurements for the patients relating to tests administered during an off-treatment period and an on-treatment period;
   (ii) identifying at least one test in said plurality of records relating to k measurements of each patient during an off-treatment period;
   (iii) identifying at least one test in said plurality of records relating to j measurements of each patient during an on-treatment period;
   (iv) identifying a best measurement from said k measurements of each patient during said off-treatment period;
   (v) performing a statistical distribution assessment on said plurality of records to identify likelihoods of a plurality of subsets of said j on-treatment measurements exceeding said best measurement, wherein the statistical distribution assessment uses a computer model based on random number generation or is derived from executing a range disparity distribution;
   (vi) selecting a threshold likelihood, corresponding to the likelihood of a number of j measurements exceeding said best measurement, said number of measurements exceeding said best measurement indicating an improved performance and thereby a treatment response in a patient;

(vii) identifying one or more acceptable subsets from the plurality of subsets of j on-treatment measurements with respective likelihoods that are not above the threshold likelihood;

(viii) comparing said j on-treatment measurements with said best measurement; and

(ix) selecting the patients who have the number of j measurements exceeding said best measurement as one of the acceptable subsets.

7.  The computer program of claim 6, wherein the patients are afflicted by a neurological disorder.

8.  The computer program of claim 7, wherein the neurological disorder is multiple sclerosis, spinal cord injuries, Alzheimer's disease or ALS.

9.  The computer program of claim 8, said patients being afflicted by a neurological disorder which is multiple sclerosis and wherein the instructions contained by said computer readable medium included with said computer program, further comprise:

    establishing a baseline comparability among groups of said patients who exhibited improved response by analyzing baseline demographic variables, key neurological characteristics, and efficacy variables at said baseline.

10. A computer configured to execute the computer program of claim 6, said computer comprising:

    a memory module for storing patient measurements, and for storing at least a first set of instructions relating to the inputting and analyzing of said patient measurements, and a second set of instructions for outputting responder information from said patient measurements;

    a central processing unit for executing said first and second set of instructions, and for outputting the responder information resulting from said executing of said first and second instructions, said central processing unit being connected to said memory module, and in operative control of said memory module; and

    an output module connected to said central processing unit for displaying said responder information.

11. The computer of claim 10, wherein the patients are afflicted by a neurological disorder.

12. The computer of claim 11, wherein the neurological disorder is multiple sclerosis, spinal cord injuries, Alzheimer's disease or ALS.

13. The computer of claim 12, said patients being afflicted by a neurological disorder which is multiple sclerosis and wherein said central processing unit further comprises:

    means for establishing a baseline comparability among groups of said patients who exhibited improved response by analyzing baseline demographic variables, key neurological characteristics, and efficacy variables at said baseline.

14. The computer implemented method of any of claims 1 to 5, wherein performing the statistical distribution assessment comprises

    determining, using a random number generation model, a probability of each of said j on-treatment measurements exceeding said best measurement; and wherein the random number generation model generates a plurality of strings of j+k random numbers within a preset range and tests a frequency at which j on-treatment measurements set exceed the best measurement.

**Patentansprüche**

1.  Computerimplementiertes Verfahren zum Analysieren der therapeutischen Wirkung einer Behandlung von Patienten in einem klinischen Umfeld, umfassend die folgenden Schritte:

    (i) Identifizieren einer Vielzahl von Datensätzen, die Patienten in einer klinischen Datenbank betreffen, wobei die Datensätze Messungen für die Patienten umfassen, die Tests betreffen, die während eines Zeitraums außerhalb der Behandlung und eines Zeitraums während der Behandlung verabreicht wurden;

    (ii) Identifizieren von mindestens einem Test in der Vielzahl der Datensätze, die k Messungen jedes Patienten während eines Zeitraums außerhalb der Behandlung betreffen;

(iii) Identifizieren von mindestens einem Test in der Vielzahl der Datensätze, die j Messungen jedes Patienten während eines Zeitraums während der Behandlung betreffen;

(iv) Identifizieren einer besten Messung aus den k Messungen jedes Patienten während des Zeitraums außerhalb der Behandlung;

(v) Durchführen einer statistischen Verteilungsauswertung mit der Vielzahl der Datensätze, um Wahrscheinlichkeiten zu identifizieren, dass eine Vielzahl von Teilmengen der j Messungen während der Behandlung die beste Messung übertreffen, wobei die statistische Verteilungsauswertung ein Computermodell verwendet, das auf Zufallszahlgenerierung basiert oder von der Ausführung einer Bereichsdisparitätsverteilung abgeleitet ist;

(vi) Auswählen einer Schwellenwertwahrscheinlichkeit, die der Wahrscheinlichkeit entspricht, dass eine Anzahl von j Messungen die beste Messung übertrifft, wobei die Anzahl der Messungen, die die beste Messung übertrifft, eine verbesserte Leistung und damit ein Ansprechen der Behandlung bei einem Patienten angibt;

(vii) Identifizieren von einer oder mehreren annehmbaren Teilmengen aus der Vielzahl der Teilmengen von j Messungen während der Behandlung mit jeweiligen Wahrscheinlichkeiten, die nicht oberhalb der Schwellenwertwahrscheinlichkeit liegen;

(viii) Vergleichen der j Messungen während der Behandlung mit der besten Messung; und

(ix) Auswählen der Patienten, bei denen die Anzahl von j Messungen die beste Messung übertrifft, als eine von den annehmbaren Teilmengen.

2. Computerimplementiertes Verfahren nach Anspruch 1, ferner umfassend Verabreichen des Tests an ausgewählte Patienten nach dem Behandlungszeitraum, wobei die Patienten basierend darauf ausgewählt werden, dass sie des Weiteren eine verbesserte Leistung während eines überwiegenden Teils der Tests zeigen, die während des Behandlungszeitraums verabreicht wurden, verglichen mit den Tests, die nach dem Behandlungszeitraum verabreicht wurden.

3. Computerimplementiertes Verfahren nach Anspruch 1 oder Anspruch 2, wobei die Patienten unter einer neurologischen Erkrankung leiden.

4. Computerimplementiertes Verfahren nach Anspruch 3, wobei die neurologische Erkrankung multiple Sklerose, Rückenmarkverletzungen, Morbus Alzheimer oder ALS ist.

5. Computerimplementiertes Verfahren nach Anspruch 4, wobei die neurologische Erkrankung multiple Sklerose ist, und das computerimplementierte Verfahren ferner den Schritt des Herstellens einer Baseline-Vergleichbarkeit innerhalb von Gruppen der Patienten umfasst, die verbessertes Ansprechen gezeigt haben, indem demographische Baseline-Variablen, neurologische Schlüsselcharakteristika und Effizienzvariablen an der Baseline analysiert werden.

6. Computerprogramm zur Verwendung mit einem Computersystem zum Analysieren der therapeutischen Wirkung einer Behandlung von Patienten in einem klinischen Umfeld, wobei das Computerprogramm umfasst:
ein computerlesbares Medium, das darauf Anweisungen enthält, die funktional sind, um den Betrieb eines Computers zur Durchführung der folgenden Schritte zu steuern:

(i) Identifizieren einer Vielzahl von Datensätzen, die Patienten in einer klinischen Datenbank betreffen, wobei die Datensätze Messungen für die Patienten umfassen, die Tests betreffen, die während eines Zeitraums außerhalb der Behandlung und eines Zeitraums während der Behandlung durchgeführt wurden;

(ii) Identifizieren von mindestens einem Test in der Vielzahl der Datensätze, die k Messungen jedes Patienten während eines Zeitraums außerhalb der Behandlung betreffen;

(iii) Identifizieren von mindestens einem Test in der Vielzahl der Datensätze, die j Messungen jedes Patienten während eines Zeitraums während der Behandlung betreffen;

(iv) Identifizieren einer besten Messung aus den k Messungen jedes Patienten während des Zeitraums außerhalb der Behandlung;

(v) Durchführen einer statistischen Verteilungsauswertung mit der Vielzahl der Datensätze, um Wahrscheinlichkeiten zu identifizieren, dass eine Vielzahl von Teilmengen der j Messungen während der Behandlung die beste Messung übertrifft, wobei die statistische Verteilungsauswertung ein Computermodell verwendet, das auf Zufallszahlgenerierung basiert oder von der Ausführung einer Bereichsdisparitätsverteilung abgeleitet ist;

(vi) Auswählen einer Schwellenwertwahrscheinlichkeit, die der Wahrscheinlichkeit entspricht, dass eine Anzahl von j Messungen die beste Messung übertrifft, wobei die Anzahl der Messungen, die die beste Messung übertrifft, eine verbesserte Leistung und damit ein Ansprechen der Behandlung bei einem Patienten angibt;

(vii) Identifizieren von einer oder mehreren annehmbaren Teilmengen aus der Vielzahl der Teilmengen von j Messungen während der Behandlung mit jeweiligen Wahrscheinlichkeiten, die nicht oberhalb der Schwellenwertwahrscheinlichkeit liegen;

(viii) Vergleichen der j Messungen während der Behandlung mit der besten Messung; und

(ix) Auswählen der Patienten, bei denen die Anzahl von j Messungen die beste Messung übertrifft, als eine von den annehmbaren Teilmengen.

7. Computerprogramm nach Anspruch 6, wobei die Patienten unter einer neurologischen Erkrankung leiden.

8. Computerprogramm nach Anspruch 7, wobei die neurologische Erkrankung multiple Sklerose, Rückenmarkverletzungen, Morbus Alzheimer oder ALS ist.

9. Computerprogramm nach Anspruch 8, wobei die Patienten unter einer neurologischen Erkrankung leiden, welche multiple Sklerose ist, und wobei die Anweisungen, die in das Computerprogramm eingeschlossen sind, das in dem computerlesbaren Medium enthalten ist, des Weiteren umfassen:

Herstellen einer Baseline-Vergleichbarkeit innerhalb von Gruppen der Patienten, die verbessertes Ansprechen gezeigt haben, indem demographische Baseline-Variablen, neurologische Schlüsselcharakteristika und Effizienzvariablen an der Baseline analysiert werden.

10. Computer, der konfiguriert ist, um das Computerprogramm gemäß Anspruch 6 auszuführen, wobei der Computer umfasst:

ein Speichermodul zum Speichern von Patientenmessungen, und zum Speichern von mindestens einer ersten Menge von Anweisungen, die das Eingeben und Analysieren der Patientenmessungen betreffen, und von einer zweiten Menge von Anweisungen zum Ausgeben von Responder-Informationen aus den Patientenmessungen; eine zentrale Verarbeitungseinheit zum Ausführen der ersten und zweiten Menge von Anweisungen, und zum Ausgeben der Responder-Informationen, die aus der Ausführung der ersten und zweiten Anweisungen resultieren, wobei die zentrale Verarbeitungseinheit mit dem Speichermodul verbunden ist und in funktionaler Steuerung des Speichermoduls ist; und ein Ausgabemodul, das mit der zentralen Verarbeitungseinheit verbunden ist, um die Responder-Informationen anzuzeigen.

11. Computer nach Anspruch 10, wobei die Patienten unter einer neurologischen Erkrankung leiden.

12. Computer nach Anspruch 11, wobei die neurologische Erkrankung multiple Sklerose, Rückenmarkverletzungen, Morbus Alzheimer oder ALS ist.

13. Computer nach Anspruch 12, wobei die Patienten an einer neurologischen Erkrankung leiden, die multiple Sklerose ist, und wobei die zentrale Verarbeitungseinheit des Weiteren umfasst:

Mittel zum Herstellen einer Baseline-Vergleichbarkeit innerhalb von Gruppen der Patienten, die verbessertes Ansprechen gezeigt haben, indem demographische Baseline-Variablen, neurologische Schlüsselcharakteristika und Effizienzvariablen an der Baseline analysiert werden.

14. Computerimplementiertes Verfahren nach einem der Ansprüche 1 bis 5, wobei das Durchführen der statistischen Verteilungsauswertung umfasst:

Ermitteln einer Wahrscheinlichkeit von jeder der j Messungen während der Behandlung, die beste Messung zu übertreffen, unter Verwendung eines Zufallszahlengenerierungsmodells; und wobei das Zufallszahlengenerierungsmodell eine Vielzahl von Ketten von j+k Zufallszahlen innerhalb eines vorgewählten Bereichs generiert und eine Häufigkeit testet, mit der j Messungsmengen während der Behandlung die beste Messung übertreffen.

## Revendications

1. Procédé mis en oeuvre par ordinateur pour analyser l'effet thérapeutique d'un traitement de patients dans un environnement clinique comprenant les étapes suivantes consistant à :

(i) identifier une pluralité d'enregistrements concernant des patients dans une base de données clinique, lesdits enregistrements comprenant des mesures pour les patients relatives à des tests administrés pendant une période sans traitement et une période de traitement ;

(ii) identifier au moins un test dans ladite pluralité d'enregistrements concernant des mesures k sur chaque patient pendant une période sans traitement ;

(ii) identifier au moins un test dans ladite pluralité d'enregistrements concernant des mesures j sur chaque patient pendant une période de traitement ;

(iv) identifier une meilleure mesure parmi lesdites mesures k sur chaque patient pendant ladite période sans traitement ;

(v) effectuer une évaluation de la distribution statistique sur ladite pluralité d'enregistrements pour identifier les probabilités d'une pluralité de sous-ensembles desdites mesures j pendant une période de traitement dépassant ladite meilleure mesure, dans lequel l'évaluation de la distribution statistique utilise un modèle informatique basé sur la génération de nombres aléatoires ou est dérivée de l'exécution d'une distribution de disparité de gamme ;

(vi) sélectionner un seuil de vraisemblance, correspondant à la probabilité qu'un nombre de mesures j dépasse ladite meilleure mesure, le fait que ledit nombre de mesures dépasse ladite meilleure mesure indiquant une performance améliorée et par conséquent une réponse au traitement chez un patient ;

(vii) identifier un ou plusieurs sous-ensembles acceptables parmi la pluralité de sous-ensembles de mesures j pendant une période de traitement avec des probabilités respectives ne dépassant pas le seuil de vraisemblance ;

(viii) comparer lesdites mesures j pendant une période de traitement avec ladite meilleure mesure ; et

(ix) sélectionner les patients pour lesquels le nombre de mesures j dépassant ladite meilleure mesure constitue l'un des sous-ensembles acceptables.

2. Procédé mis en oeuvre par ordinateur selon la revendication 1, comprenant en outre l'étape consistant à administrer le test à des patients sélectionnés après la période de traitement, dans lequel les patients sont sélectionnés sur la base de la présentation supplémentaire d'une performance améliorée au cours d'une majorité des tests administrés pendant la période de traitement par rapport au test administré après la période de traitement.

3. Procédé mis en oeuvre par ordinateur selon la revendication 1 ou la revendication 2, dans lequel les patients sont atteints d'un trouble neurologique.

4. Procédé mis en oeuvre par ordinateur selon la revendication 3, dans lequel le trouble neurologique est la sclérose en plaques, des lésions de la moelle épinière, la maladie d'Alzheimer ou la SLA.

5. Procédé mis en oeuvre par ordinateur selon la revendication 4, dans lequel le trouble neurologique est la sclérose en plaques et le procédé mis en oeuvre par ordinateur comprend en outre l'étape consistant à établir une comparabilité de base entre les groupes desdits patients qui ont présenté une réponse améliorée en analysant les variables démographiques de la base de référence, les caractéristiques neurologiques clés et les variables d'efficacité au niveau de ladite base de référence.

6. Programme informatique destiné à être utilisé avec un système informatique pour analyser l'effet thérapeutique d'un traitement de patients dans un environnement clinique, le programme informatique comprenant :
un support lisible par ordinateur contenant des instructions permettant de contrôler le fonctionnement d'un ordinateur afin d'effectuer les étapes consistant à :

(i) identifier une pluralité d'enregistrements concernant des patients dans une base de données clinique, lesdits enregistrements comprenant des mesures pour les patients relatives à des tests administrés pendant une période sans traitement et une période de traitement ;

(ii) identifier au moins un test dans ladite pluralité d'enregistrements concernant des mesures k sur chaque patient pendant une période sans traitement ;

(ii) identifier au moins un test dans ladite pluralité d'enregistrements concernant des mesures j sur chaque patient pendant une période de traitement ;

(iv) identifier une meilleure mesure parmi lesdites mesures k sur chaque patient pendant ladite période sans traitement ;

(v) effectuer une évaluation de la distribution statistique sur ladite pluralité d'enregistrements pour identifier les probabilités d'une pluralité de sous-ensembles desdites mesures j pendant une période de traitement dépassant ladite meilleure mesure, dans lequel l'évaluation de la distribution statistique utilise un modèle informatique basé sur la génération de nombres aléatoires ou est dérivée de l'exécution d'une distribution de disparité de gamme ;

(vi) sélectionner un seuil de vraisemblance, correspondant à la probabilité qu'un nombre de mesures j dépasse ladite meilleure mesure, le fait que ledit nombre de mesures dépasse ladite meilleure mesure indiquant une performance améliorée et par conséquent une réponse au traitement chez un patient ;

(vii) identifier un ou plusieurs sous-ensembles acceptables parmi la pluralité de sous-ensembles de mesures j pendant une période de traitement avec des probabilités respectives ne dépassant pas le seuil de vraisemblance ;

(viii) comparer lesdites mesures j de traitement avec ladite meilleure mesure ; et

(ix) sélectionner les patients pour lesquels le nombre de mesures j dépassant ladite meilleure mesure constitue l'un des sous-ensembles acceptables.

7. Programme informatique selon la revendication 6, dans lequel les patients sont atteints d'un trouble neurologique.

8. Programme informatique selon la revendication 7, dans lequel le trouble neurologique est la sclérose en plaques, des lésions de la moelle épinière, la maladie d'Alzheimer ou la SLA.

9. Programme informatique selon la revendication 8, lesdits patients étant atteints d'un trouble neurologique qui est la sclérose en plaques et dans lequel les instructions contenues dans ledit support lisible par ordinateur inclus avec ledit programme d'ordinateur comprennent en outre celles permettant :
d'établir une comparabilité de base entre les groupes desdits patients qui ont présenté une réponse améliorée en analysant les variables démographiques de la base de référence, les caractéristiques neurologiques clés et les variables d'efficacité au niveau de ladite base de référence.

10. Ordinateur configuré pour exécuter le programme d'ordinateur selon la revendication 6, ledit ordinateur comprenant :

un module de mémoire pour stocker des mesures sur le patient et pour stocker au moins un premier ensemble d'instructions relatives à la saisie et à l'analyse desdites mesures sur le patient, et un second ensemble d'instructions pour délivrer des informations de réponse à partir desdites mesures sur le patient ;
une unité centrale de traitement pour exécuter lesdits premier et second ensembles d'instructions et pour délivrer les informations de réponse résultant de ladite exécution desdites première et seconde instructions, ladite unité centrale de traitement étant connectée audit module de mémoire, et sous le contrôle opérationnel dudit module de mémoire ; et
un module de sortie connecté à ladite unité centrale de traitement pour afficher lesdites informations de réponse.

11. Ordinateur selon la revendication 10, dans lequel les patients sont atteints d'un trouble neurologique.

12. Ordinateur selon la revendication 11, dans lequel le trouble neurologique est la sclérose en plaques, des lésions de la moelle épinière, la maladie d'Alzheimer ou la SLA.

13. Ordinateur selon la revendication 12, lesdits patients étant atteints d'un trouble neurologique qui est la sclérose en plaques et dans lequel ladite unité centrale de traitement comprend en outre :
des moyens pour établir une comparabilité de base entre les groupes desdits patients qui ont présenté une réponse améliorée en analysant les variables démographiques de la base de référence, les caractéristiques neurologiques clés et les variables d'efficacité au niveau de ladite base de référence.

14. Procédé mis en oeuvre par ordinateur selon l'une quelconque des revendications 1 à 5, dans lequel l'exécution de l'évaluation de la distribution statistique comprend les étapes consistant à
déterminer, en utilisant un modèle de génération de nombres aléatoires, une probabilité que chacune desdites mesures j pendant une période de traitement dépasse ladite meilleure mesure ; et dans lequel le modèle de génération de nombres aléatoires génère une pluralité de chaînes de nombres aléatoires j+k dans une plage prédéfinie et teste une fréquence à laquelle les mesures j pendant une période de traitement dépassent la meilleure mesure.

**Fig. 1**

START

Input values *(k)* of all patient performances of all patients during *(x)* time period of Off-Treatment.    100

Select Best Measurement from each given patient *(P)* of all patients during *(x)* time period of his Off-Treatment.  104

Flag Best Measurement; Set Best Measurement Performance Value *(k_f)* as his own Comparative Baseline.    108

Input Values *(j)* (where values *j* consists of active *(j_a)* and placebo *(j_p)*) for each of the Patient Performances during *(y)* time period of On-Treatment.                    110

Generate Probability Distribution for the number of theoretical values of *j (j_t)*, based on parallel *x*, *y*, and *P* values, that are statistically likely to exceed the maximum theoretical value of *k (k_tf)* where the theoretical value is that predicted by a model based on random variability.                114

(optional)

Reset *P*, *x*, *y*

Yes

Is probability of majority of [values *(j_t)*>values *(k_tf)*] greater than an acceptable placebo response rate, say 10%? 118

No

Determine Number of Values *(j_a)* > Values *(k_fa)*; Determine Number of Values *(j_p)* > *(k_fp)*        122

Compare Distribution of number of values *(j_a)* > values *(k_fa)* with distribution of number of values *(j_p)* > values *(k_fp)*   126

(optional)

Reset *P*, *x*, *y*

No

Is distribution of *(j_a)*>values *(k_fa)* significantly higher than distribution of values

Yes

END

**Fig. 2**

```
                    ┌─────────────────────┐
                    (        START         )
                    └─────────────────────┘
                               │
                               ▼
         ┌───────────────────────────────────────────┐
         │  Set Range of Values (j), (k)     202     │
         │                                           │
         └───────────────────────────────────────────┘
                               │
                               ▼
         ┌───────────────────────────────────────────┐
         │  Generate random values for (j), (k)      │
         │  for (z) number of sets.        206       │
         └───────────────────────────────────────────┘
                               │
                               ▼
         ┌───────────────────────────────────────────┐
         │  Determine probabilities for each         │
         │  respective integer within entire range of│
         │  values (j) to exceed the maximum         │
         │  value (k) in a given set.       210      │
         └───────────────────────────────────────────┘
                               │
                               ▼
         ┌───────────────────────────────────────────┐
         │  Output probability distribution.   214   │
         └───────────────────────────────────────────┘
                               │
                               ▼
                    ┌─────────────────────┐
                    (         END          )
                    └─────────────────────┘
```

**Fig. 3**

Clinical Trial
Measurements-
Off Drug <u>504</u>

Clinical Trial
Measurements-
On Drug <u>508</u>

Clinical
Database
<u>510</u>

Treatment Response Detector
(processing and output) <u>514</u>

Statistical
Generator <u>518</u>

(PC based)

Figure 4 (a)

**Figure 4 (b)**

**Figure 4(c)**

Figure 4(d)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02051354 A2
- US 2005075832 A1

- US 11102559 B


**Non-patent literature cited in the description**

- **BLIGHT, A. R.** Effect of 4-aminopyridine on axonal conduction block in chronic spinal cord injury. *Brain Res Bull,* 1989, vol. 22, 47-52
- **WAXMAN SG.** Aminopyridines and the treatment of spinal cord injury. *J Neurotrauma,* 1993, vol. 10, 19-24
- **DAVIS, F.A. ; D. STEFOSKI ; J. RUSH.** Orally administered 4-aminopyridine improves clinical signs in multiple sclerosis. *Ann Neurol,* 1990, vol. 27, 186-192
- **VAN DIEMEN, H.A.M. et al.** The effect of 4-aminopyridine on clinical signs in multiple sclerosis: a randomized, placebo-controlled, double-blind, crossover study. *Ann Neurol,* 1992, vol. 32, 123-130

- **SCHWID, S.R. et al.** Quantitative assessment of sustained-release 4-aminopyridine for symptomatic treatment of multiple sclerosis. *Neurology,* 1997, vol. 48, 817-821
- **MCDONALD, W.I. et al.** Recommended Diagnostic Criteria for Multiple Sclerosis: Guidelines from the International Panel on the Diagnosis of Multiple Sclerosis. *Ann Neurol,* 2001, vol. 50, 121-127
- **HOBART J ; RIAZI A ; LAMPLING D ; FITZPATRICK R ; THOMPSON A.** Measuring the Impact of MS on Walking Ability. *J Neurol,* 2003, vol. 60, 31-36
- Presented at the 130th Annual Meeting. American Neurological Association, 25 September 2005